(19) Europäisches Patentamt
European Patent Office
Office européen des brevets

(11) **EP 2 650 355 A1**

(12) **EUROPEAN PATENT APPLICATION**

(43) Date of publication:
**16.10.2013 Bulletin 2013/42**

(51) Int Cl.:
**C12C 3/08** *(2006.01)*    **C12C 3/12** *(2006.01)*
**C12P 7/06** *(2006.01)*

(21) Application number: **13175586.0**

(22) Date of filing: **09.02.2004**

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **10.02.2003 US 361976**

(62) Document number(s) of the earlier application(s) in accordance with Art. 76 EPC:
**04709423.0 / 1 611 230**

(71) Applicant: **John I. Haas, Inc.**
**Washington, DC 20016-3341 (US)**

(72) Inventor: **Maye, John, Paul**
**Washington, DC District of Columbia 20016-3341 (US)**

(74) Representative: **Stippl, Hubert**
**STIPPL Patentanwälte**
**Freiligrathstrasse 7a**
**90482 Nürnberg (DE)**

Remarks:
This application was filed on 08-07-2013 as a divisional application to the application mentioned under INID code 62.

(54) **A method for controlling lactic acid bacteria contamination in a process medium used in the production of fuel ethanol**

(57)     A method for controlling lactic acid bacteria contamination in a process medium used in the production of fuel ethanol comprising:

adding an aqueous alkaline solution of hop acid to a process medium having a pH less than the pH of the alkaline hop acid solution,

wherein the hop acid is selected from at least one of the group consisting of alpha acids, beta acids, isoalpha acids, rho-isoalpha acids, tetrahydroisoalpha acids and hexahydroisoalpha acids and salts thereof.

Fig. 46

EP 2 650 355 A1

**Description**

BACKGROUND

The present invention relates to an improved process for controlling micro-organisms in an aqueous process medium by using hop acids. The present invention further relates to the manufacture of fuel ethanol. More particularly, it relates to a process for the production of fuel ethanol using hop acids.

There exists in the world today an enormous demand for liquid fuels and this is being supplied almost entirely by distilled petroleum oils. It is, of course, well known that petroleum is a non-renewable resource and that finite supplies of this fuel source exist. As a result, there is now a very active search for alternative liquid fuels or fuel extenders.

In light of the steadily increasing demand for liquid fuels and the shrinking resources for petroleum crude oil, researchers have begun to investigate alternative liquid fuels to determine the feasibility of commercially producing such substitutes in order to fulfill this increasing demand. Recent world events, including the shortage of petroleum crude oil, the sharp increase in the cost of oil and gasoline products, and the political instability of many oil-producing countries, have demonstrated the vulnerability of the present sources of liquid fuels. Even if such supply and economic instabilities were acceptable, it is clear that the worldwide production of petroleum products at forecasted levels can neither keep pace with the increasing demand nor continue indefinitely. It is becoming evident that the time will soon come when there will have to be a transition to resources which are plentiful and preferably renewable.

One of the most generally recognized substitutes which could be made available in significant quantities in the near future is alcohol, and in particular, ethanol. For example, there are currently many outlets in the United States and throughout the world which sell a blend of gasoline and about 10 percent to 20 percent ethanol (commonly called "gasohol") which can be used as a fuel in conventional automobile engines. Furthermore, ethanol can be blended with additives to produce a liquid ethanol-based fuel, with ethanol as the major component, which is suitable for operation in most types of engines.

Ethanol can be produced from almost any material which either exists in the form of, or can be converted into, a fermentable sugar. There are many natural sugars available for fermentation, but carbohydrates such as starch and cellulose can be converted into fermentable sugars which then ferment into ethanol. Even today, throughout most of the world, ethanol is produced through the fermentation process. Ethanol can also be produced synthetically from ethylene.

Starch is one of the world's most abundant renewable raw materials. One answer to the need for alternative reproducible fuels is to convert this very abundant material at low cost into fermentable sugars as feedstock for fermentation to ethanol. A process medium used in the production of fuel ethanol is intended to be an inclusive term encompassing any of the mediums in which lactic acid or acetic acid bacteria can live and used in the production of fuel ethanol or spirits and includes, but is not limited to, feedstock, any saccharified or hydrolysised starch or sugar medium, any starch or sugar medium including yeast, and/or the distillate from any fermentation process. The starch for the feedstock process usually comes from crops such as corn, milo, wheat, malted barley, potatoes and rice. The fermentable sugars obtained from starch are glucose and maltose and these are typically obtained from the starch by hydrolysis or saccharification, e.g. acid hydrolysis or enzyme hydrolysis. Most hydrolysis techniques which have been available have tended to be very expensive in terms of producing a feedstock for large scale alcohol production. In terms of maximizing ethanol production from a starch raw material source, it is desirable to have the fermentables as high as possible in the fermentation substrate.

Experience has taught that it is preferable to add malt enzymes, such as glucoamylase, which aid in the hydrolysis of starches and conversion of the higher complex dextrin and dextrose sugars which are present in the sugar solutions of the prior art fermentation processes. Malt enzymes can be purchased, or in the case of whiskey production, extracted naturally from malted barley. While such malt enzymes add a desirable flavor to ethanol produced for human consumption, the malt enzymes do not make ethanol a more advantageous liquid fuel substitute and, in fact, could create problems for such a use.

After the saccharification step is completed, the fermentable sugars are added to yeast where fermentation begins. Alternatively, today many distillers add the enzyme to the fermenter with the yeast. This simultaneous saccharification and fermentation allows for higher concentrations of starch to be fermented. If the sugar source comes from crops such as sugar cane, sugar beets, fruit or molasses, saccharification is not necessary and fermentation can begin with the addition of yeast and water.

With the typical known systems for producing ethanol from starch, e.g. using a dual enzyme system for liquefying and saccharifying the starch to glucose followed by batch fermentation, total processing times of 60 to 80 hours are usual. Fermentation times of 50 to 70 hours are commonplace. Such long total residence times result in enormous tankage requirements within the processing system when large scale ethanol production is contemplated.

In the fermentation process, yeast is added to a solution of simple sugars. Yeast is a small microorganism which uses the sugar in the solution as food, and in doing so, expels ethanol and carbon dioxide as byproducts. The carbon dioxide comes off as a gas, bubbling up through the liquid, and the ethanol stays in solution. Unfortunately, the yeast stagnate when the concentration of the ethanol in solution approaches about 18 percent by volume, whether or not there are still fermentable sugars present.

In order for nearly complete fermentation, and in order to produce large quantities of ethanol, the common practice has been to use a batch process wherein extremely large fermentation vessels capable of holding upwards of 500,000 gallons are used. With such large vessels, it is economically unrealistic to provide an amount of yeast sufficient to rapidly ferment the sugar solution. Hence, conventional fermentation processes have required 72 hours and more because such time periods are required for the yeast population to build to the necessary concentration. For example, a quantity of yeast is added to the fermentation vessel. In approximately 45-60 minutes, the yeast population will have doubled; in another 45-60 minutes that new yeast population will have doubled. It takes many hours of such propagation to produce the quantity of yeast necessary to ferment such a large quantity of sugar solution.

The sugars used in traditional fermentation processes have typically contained from about 6 percent to 20 percent of the larger, complex sugars, such as dextrins and dextrose, which take a much longer time to undergo fermentation, if they will undergo fermentation, than do the simple hexose sugars, such as glucose and fructose. Thus, it is common practice to terminate the fermentation process after a specified period, such as 72 hours, even though not all of the sugars have been utilized. Viewing the prior art processes from an economic standpoint, it is preferable to sacrifice the remaining unfermented sugars than to wait for the complete fermentation of all of the sugars in the batch.

One of the important concerns with conventional fermentation systems is the difficulty of maintaining a sterile condition free from bacteria in the large-sized batches and with the long fermentation period. Unfortunately, the optimum atmosphere for fermentation is also extremely conducive to bacterial growth. Should a batch become contaminated, not only must the yeast and sugar solution be discarded, but the entire fermentation vessel must be emptied, cleaned, and sterilized. Such an occurrence is both time-consuming and very costly.

Additionally, many of these bacteria compete with the yeast for sugar, thereby reducing the amount of ethanol that is produced. Bacteria can grow nearly ten times faster than yeast, thus contamination in these areas are inevitable. Upon the consumption of sugar, these bacteria produce lactic acid and other byproducts. Further, if the fermentation vessels are not properly disinfected or sterilized between batches or uses, bacteria and other undesirable microorganisms can become attached to the interior walls of the fermentation vats where they will grow and flourish. These undesirable microorganisms may contaminate ethanol co- products such as animal feed, or they may consume valuable quantities of the substrate, or sugar, thus reducing the production of ethanol. The economics and efficiency of fermentation processes are frequently such that they cannot tolerate any such loss of production.

During the manufacturing of fuel ethanol, bacteria contamination occurs in nearly every step of the process where water and starch/sugar are present at temperatures below 40 °C. Contamination generally originates from the starch material since these crops pick-up bacteria from the field. Washing the material helps lower the bacteria count, however, bacteria contamination is unavoidable. An example of this is in the wet-milling processes where corn is steeped for about 24-48 hours. Just the soaking of dried corn kernels i n water generates lactic acid levels as high a s 0.5%. F or e very gram of lactic acid formed, nearly two grams of starch is lost. *Lactobacillus brevis* and *Lactobacillus fermentum* are two heterofermenter bacteria commonly found in distillery mashes. These bacteria are able to convert one mole of glucose into one mole of lactic acid and one mole of acetic acid respectively in addition to one mole of ethanol and one mole of carbon dioxide.

Current methods used to kill these unwanted microorganisms, among others, often involve introduction of foreign agents, such as antibiotics, heat, and strong chemical disinfectants, to the fermentation before or during production of ethanol. Commonly, synthetic chemical antibiotics are added to the fermentation vessels in an attempt to decrease the growth of lactic acid producing bacteria. The addition of each of these foreign agents to the process significantly adds to the time and costs of ethanol production. Antibiotics are very expensive and can add greatly to the costs of a large-scale production. If no antibiotics are used, a 1 to 5 percent loss in ethanol yield is common. A fifty million-gallon fuel ethanol plant operating with a lactic acid level of 0.3 percent weight/weight in its distiller's beer is loosing roughly 570,000 gallons of ethanol every year due to bacteria. The use of heat requires substantial energy to heat the fermentation vessels as well as possibly requiring the use of special, pressure-rated vessels that can withstand the high temperatures and pressures generated in such heat sterilizing processes. Chemical treatments can also add to the cost of production due primarily to the cost of the chemicals themselves, these chemicals are often hazardous materials requiring special handling and environmental and safety precautions, and are not "green", i.e., are not organic.

After fermentation, traditional processes have removed the ethanol from the fermentation solution and further concentrated the ethanol product by distillation. Distillation towers capable of such separation and concentration are well-known in the art. Following fermentation, the 5 to 15 percent alcoholic solution, often referred to as distiller's beer or wine, is concentrated to 50 to 95 percent ethanol via distillation. This ethanol can be used "as is" to make spirits. Alternatively, the 95 percent ethanol, generally made at fuel ethanol plants, is passed through molecular sieves to remove the remaining water to make fuel grade ethanol, greater than 99% ethanol, used for blending with gasoline.

Fuel ethanol is produced by a dry milling or wet milling process. Dry-milling starts by grinding dry corn kernels into nearly a powder, followed by cooking and treatment with high temperature enzymes to b reak down the starch into fermentable sugars. This sugary solution containing about 30 percent solids, 70 percent of which is starch, is cooled to 30 °C, treated with yeast and fermented into ethanol via batch or continuous fermentation. The ethanol is isolated from this solution via distillation. The remaining solids in this solution are isolated, dried and sold as cattle feed.

During wet-milling, dry corn kernels are steeped with water to allow the kernels to absorb moisture. The steep water is removed and the soaked kernels get loosely ground and processed through a number of steps to separate the germ, the fiber, the gluten, and the starch. The starch is processed into high fructose corn syrup, of which some gets sold to candy, food and soda companies. The remaining high fructose corn syrup is treated with yeast and fermented into ethanol.

There is much to be desired in the field of ethanol production for effective fermentation vessel sterilization that is safe, low cost, and environmentally sound, yet which enhances, rather than degrades or limits efficient alcohol producing microorganism activity. There is a need in the art for a compound and a method in which to increase fuel ethanol yields from fermentation.

Hops have been used in brewing for well over one thousand years. This pine-cone-looking ingredient is known to impart bitterness, aroma, and preservative properties to beer. Many of the active compounds responsible for bitterness are also responsible for the hop's preservative properties. These compounds have been identified and are organic acid in nature. One major compound within the hop is an organic acid known as humulone, also referred to as alpha acids. Alpha acids make-up 10 to 15 percent w/w in dry hops and over 50 percent by weight of carbon dioxide hop extract. During the brewing of beer, hops are boiled and the alpha acids undergo thermal isomerization forming a new compound known as isoalpha acids. Isoalpha acids are the actual bittering and preserving compounds found in beer.

Over the past forty years the hop industry has developed into a high-technology ingredients supplier for the brewing industry. Today hops are extracted with $CO_2$ and much of this $CO_2$ hop extract is further processed to separate the alpha acid fraction from the remainder of the hop extract. The alpha acids are then thermally isomerize into isoalpha acids and formulated to exact specifications for ease of use and precise addition to beer. Derivatives of isoalpha acids are also made by performing simple chemical reductions. These reduced isoalpha acids, specifically rho-isoalpha acids, tetrahydroisoalpha acids (THIAA) and hexahydroisoalpha acids (HHIAA) are very stable toward light and heat.

There is a need in the art for a compound and a method to reduce microorganism growth in fuel ethanol fermentation in order to increase ethanol yield.

These and other limitations and problems of the past are solved by the present invention.

BRIEF SUMMARY OF THE INVENTION

A method and compound for the reduction of lactic acid producing micro-organisms in a process medium is shown and described.

In one embodiment, when an aqueous alkaline solution of hop acid is added to a process medium having a pH less than the pH of the alkaline hop acid solution, the hop acid is especially effective at controlling micro-organisms. Indeed, the overall usage of hop acid for obtaining the desired effect can be enormously reduced. Accordingly, a process is disclosed for controlling micro-organisms in an aqueous process medium including adding an aqueous alkaline solution of a hop acid to the process medium, wherein the pH of the aqueous alkaline hop solution is higher than the pH of the process medium.

As a result of the low dosage quantity of added solution compared to the process medium, the solution adapts almost entirely the pH of the process medium when added to the process medium and the hop acid passes from the disassociated form (salt form) to the associated (free acid), anti-bacterial effective, form. Surprisingly, hop acid is especially effective as an anti-bacterial agent when used in this manner. In addition different forms of hop acids can be used which could otherwise not be used or could only be used at low effectiveness.

Isomerized hop acids are particularly effective at controlling the bacterial growth in the process mediums or streams of distilleries. Indeed, by using a standardized solution of isomerized hop acids, one is able to accurately dose the amount of hop acid required to control bacterial growth.

The invention will best be understood by reference to the following detailed description of the preferred embodiment, taken in conjunction with the accompanying drawings. The discussion below is descriptive, illustrative and exemplary and is not to be taken as limiting the scope defined by any appended claims.

BRIEF DESCRIPTION OF SEVERAL VIEWS OF THE DRAWINGS

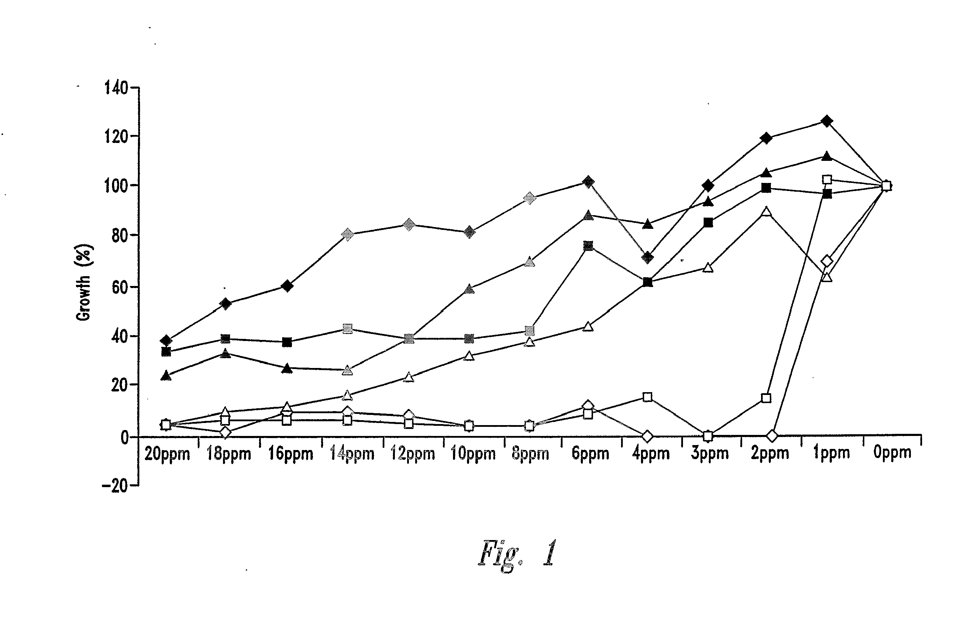

Figure 1 shows growth of *Lactobacillus brevis* LTH 5290 (*Lb. brevis*) at a range of different concentrations of various hop compounds and derivates of hop compounds in modified MRS at 86 °F. MRS medium adjusted to pH 5.2 was inoculated with *Lb*. *brevis* ($10^6$ organism/mL) After 60 hours incubation growth was assessed photometrically at 578 nm in a cell of 1 cm path length: ▲α- acids; ■ β- acids and essential oils; ♦ rho- iso- α- acids; Δ iso- α- acids; □ hexahydro- iso- α- acids; 0 tetrahydro- iso- α- acids.

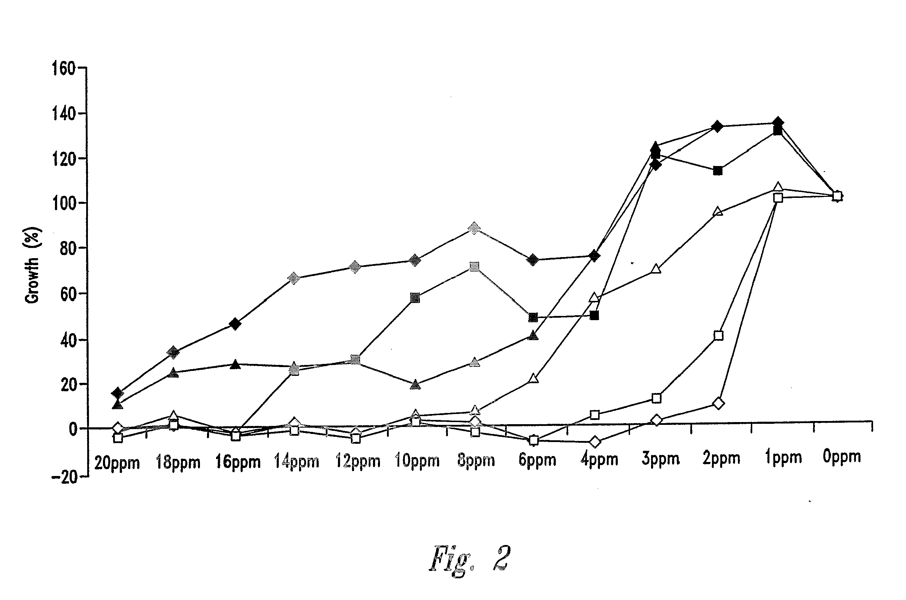

Figure 2 shows growth of *Lactobacillus fermentum* LTH 5289 (*Lb.fermentum*) at a range of different concentrations of various hop compounds and derivates of hop compounds in modified MRS at 96.8 °F. MRS medium adjusted to pH 5.2 was inoculated with *Lb.fermentum* ($10^6$ organism/mL) After 60 hours incubation growth was assessed photometrically at 578 nm in a cell of 1 cm path length: ▲a- acids; ■ β- acids and essential oils; ♦ rho- iso- α- acids; Δ iso- α- acids; □ hexahydro- iso- α- acids; 0 tetrahydro- iso- α- acids.

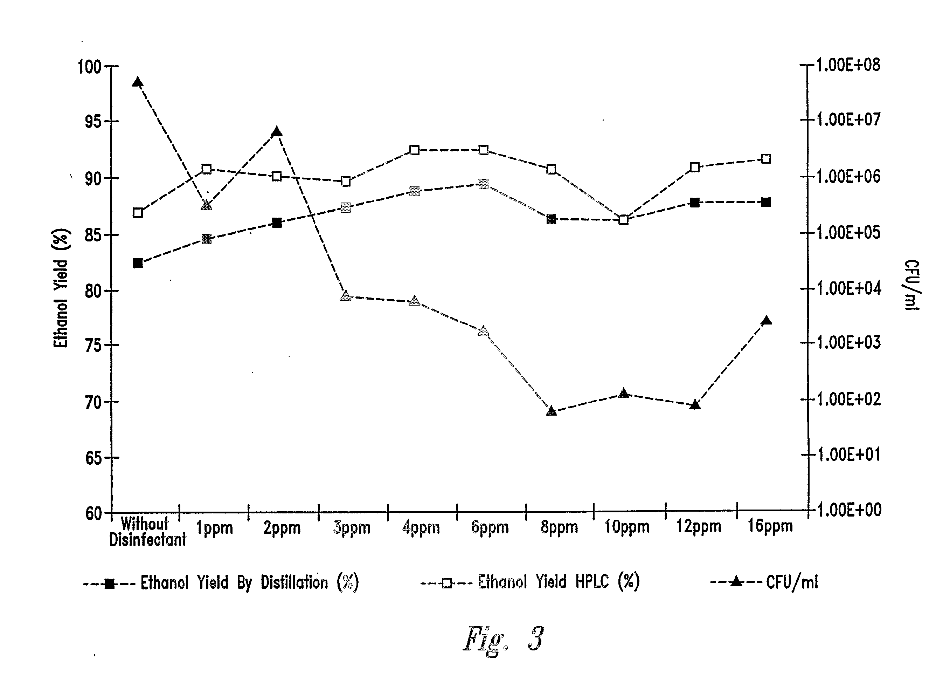

Figure 3 shows the development of ethanol yield at decreasing viable cell numbers of *Lb. brevis* correlated with increasing concentrations of tetrahydro- iso- α- acids in molasses wort. Molasses wort containing 129.74 g/L of sucrose was contaminated with initial bacterial cell numbers of $10^6$/mL. Fermentation was carried out at pH 5.2 and 86°F for 96 hours.

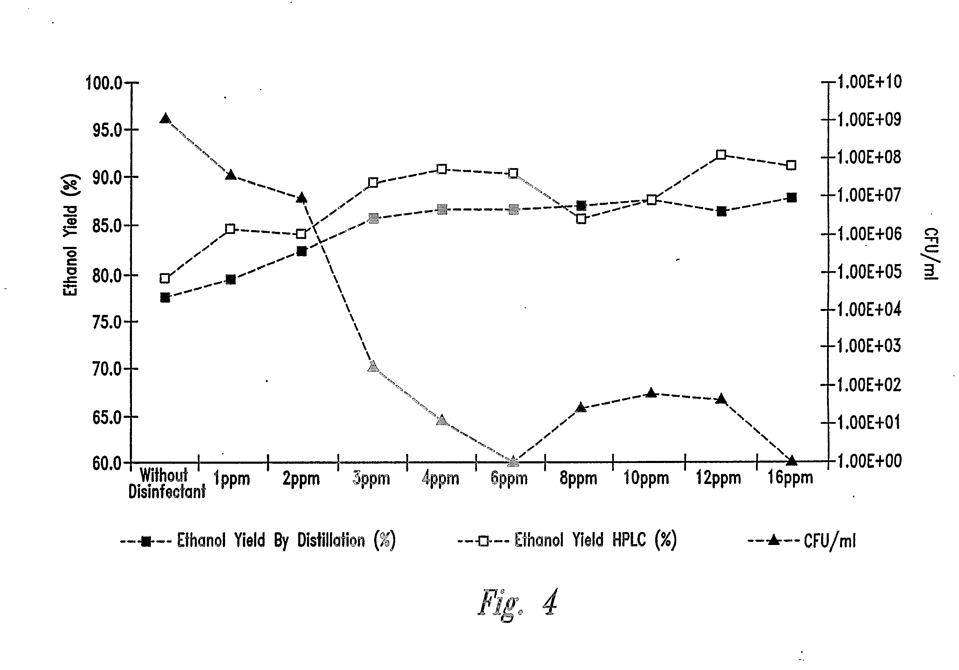

Figure 4 shows the development of ethanol yield at decreasing viable cell numbers of *Lb. fermentum* correlated with increasing concentrations of tetrahydro- iso- α- acids in molasses wort. Molasses wort containing 129.74 g/L of sucrose was contaminated with initial bacterial cell numbers of $10^6$/mL. Fermentation was carried out at pH 5.2 and 96.8°F for 72 hours.

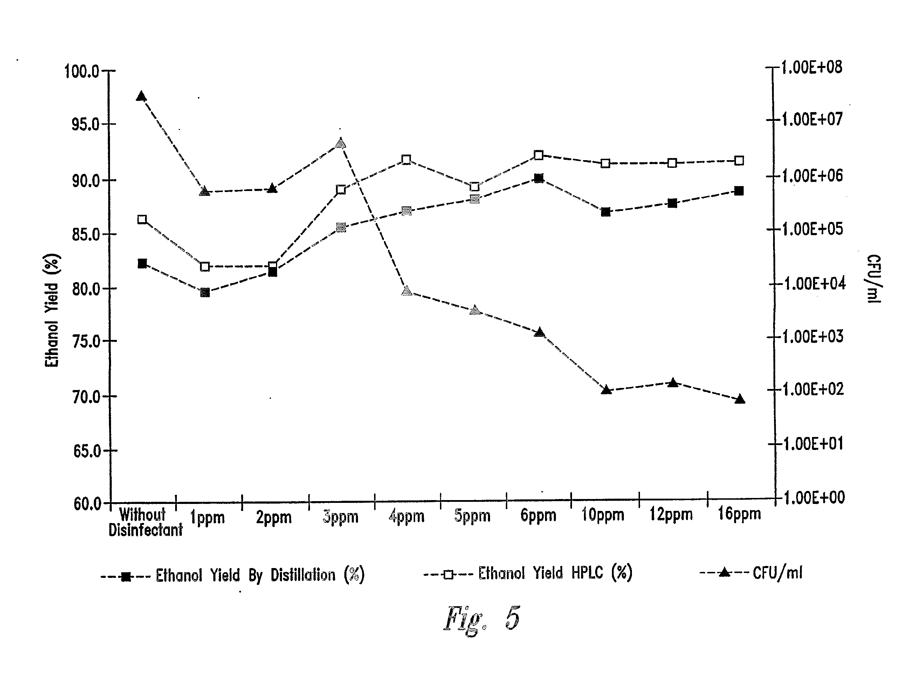

Figure 5 shows the development of ethanol yield at decreasing viable cell numbers of *Lb. brevis* correlated with increasing concentrations of hexahydro- iso- α- acids in molasses wort. Molasses wort containing 129.74 g/L of sucrose was contaminated with initial bacterial cell numbers of $10^6$/mL. Fermentation was carried out at pH 5.2 and 96.8°F for 72 hours.

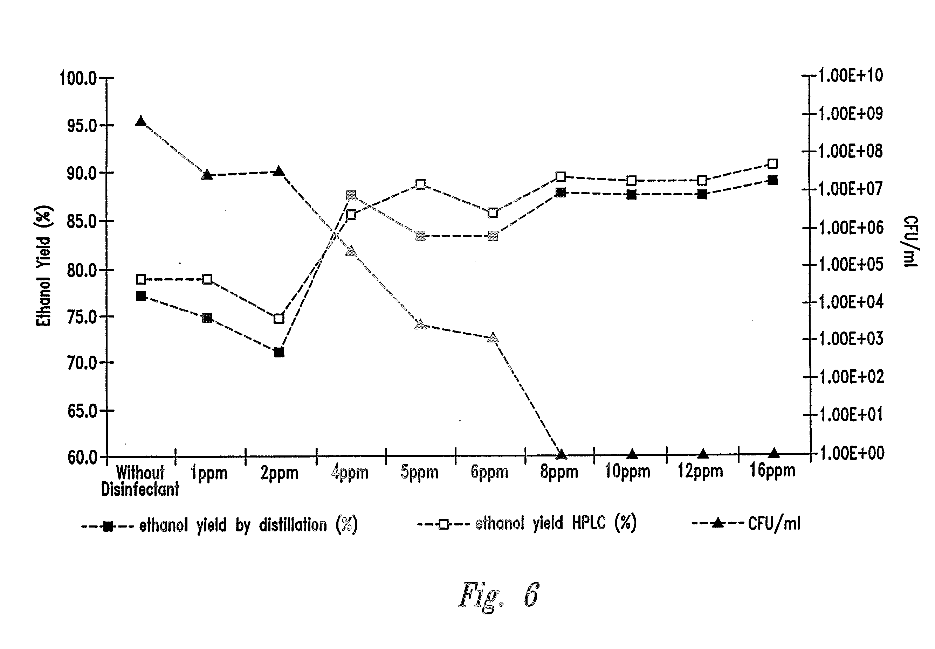

Figure 6 shows the development of ethanol yield at decreasing viable cell numbers of *Lb. fermentum* correlated with increasing concentrations of hexahydro- iso- α- acids in molasses wort. Molasses wort containing 129.74 g/L of sucrose was contaminated with initial bacterial cell numbers of $10^6$/mL. Fermentation was carried out at pH 5.2 and 96.8°F for 72 hours.

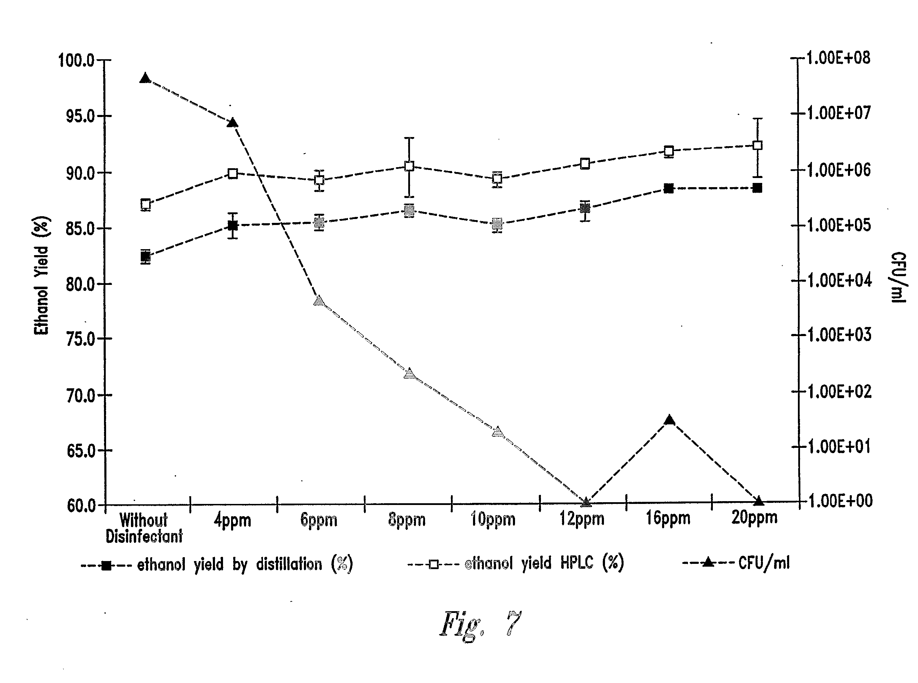

Figure 7 shows the development of ethanol yield at decreasing viable cell numbers of *Lb. brevis* correlated with increasing concentrations of iso-a-acids in molasses wort. Molasses wort containing 129.74 g/L of sucrose was contaminated with initial bacterial cell numbers of $10^6$/mL. Fermentation was carried out at pH 5.2 and 86°F for 96 hours.

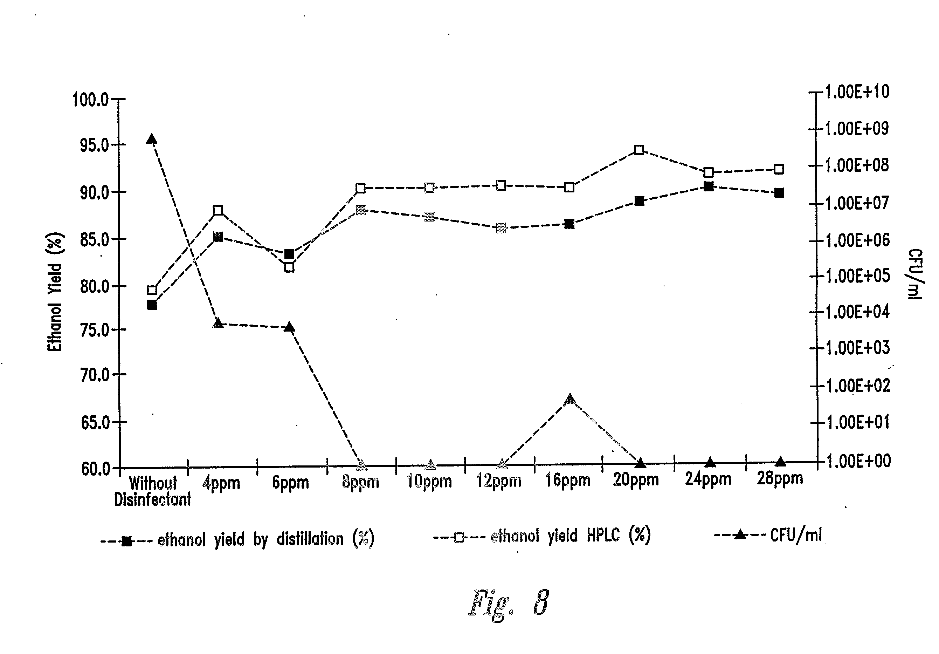

Figure 8 shows the development of ethanol yield at decreasing viable cell numbers of *Lb. fermentum* correlated with increasing concentrations of iso-a-acids in molasses wort. Molasses wort containing 129.74 g/L of sucrose was contaminated with initial bacterial cell numbers of $10^6$/mL. Fermentation was carried out at pH 5.2 and 96.8°F for 72 hours.

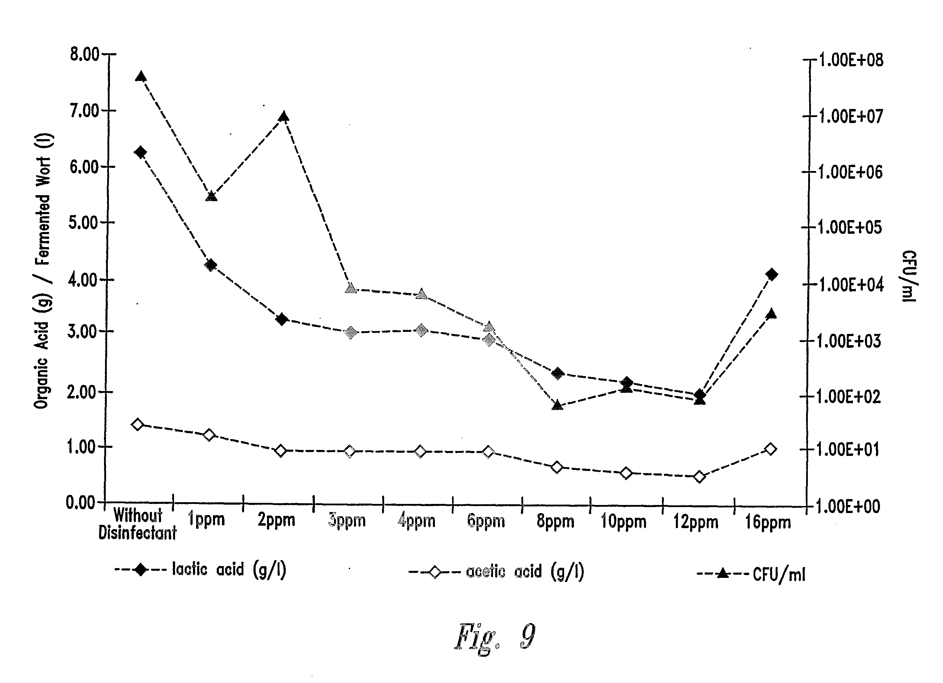

Figure 9 shows the decrease of bacterial metabolites produced by *Lb. brevis* at increasing concentrations of tetrahydro- iso- a- acids in fermented molasses wort.

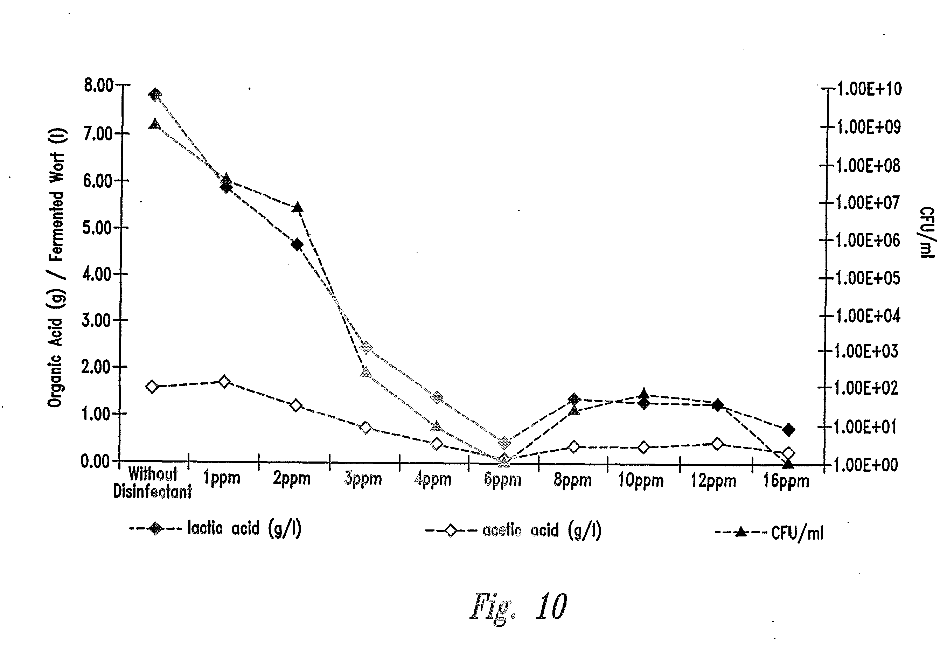

Figure 10 shows the decrease of bacterial metabolites produced by *Lb. fermentum* at increasing concentrations of tetrahydro- iso- a- acids in fermented molasses wort.

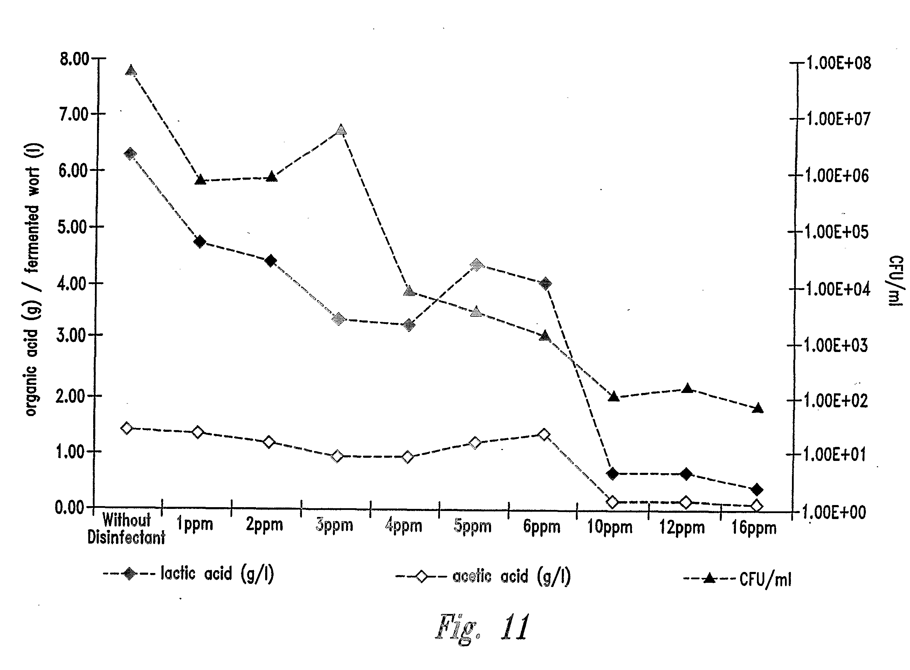

Figure 11 shows the decrease of bacterial metabolites produced by *Lb. brevis* at increasing concentrations of hexahydro- iso- a- acids in fermented molasses wort.

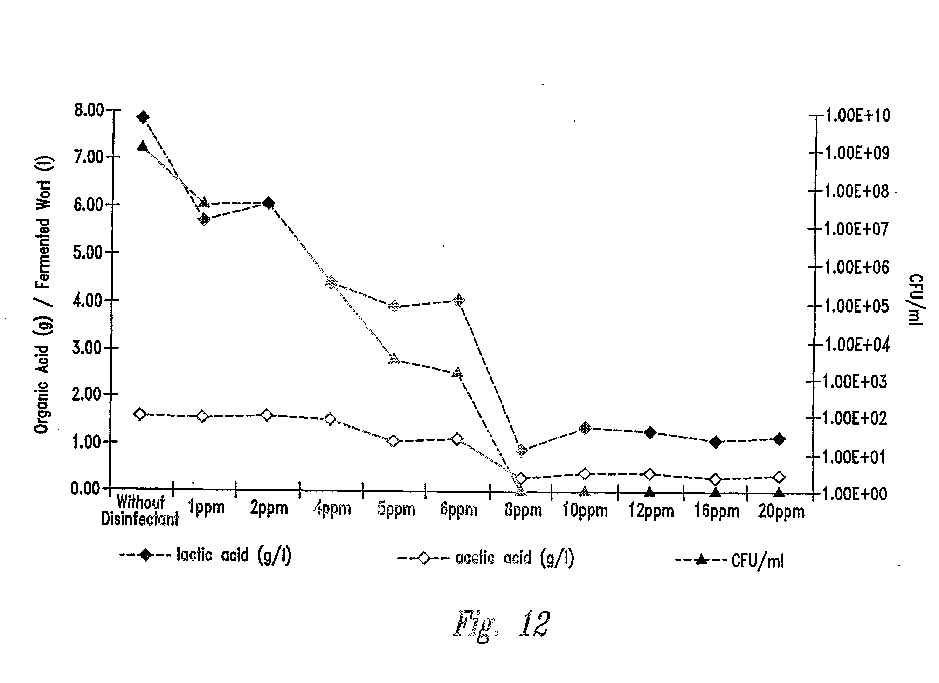

Figure 12 shows the decrease of bacterial metabolites produced by *Lb. fermentum* at increasing concentrations of hexahydro- iso- a- acids in fermented molasses wort.

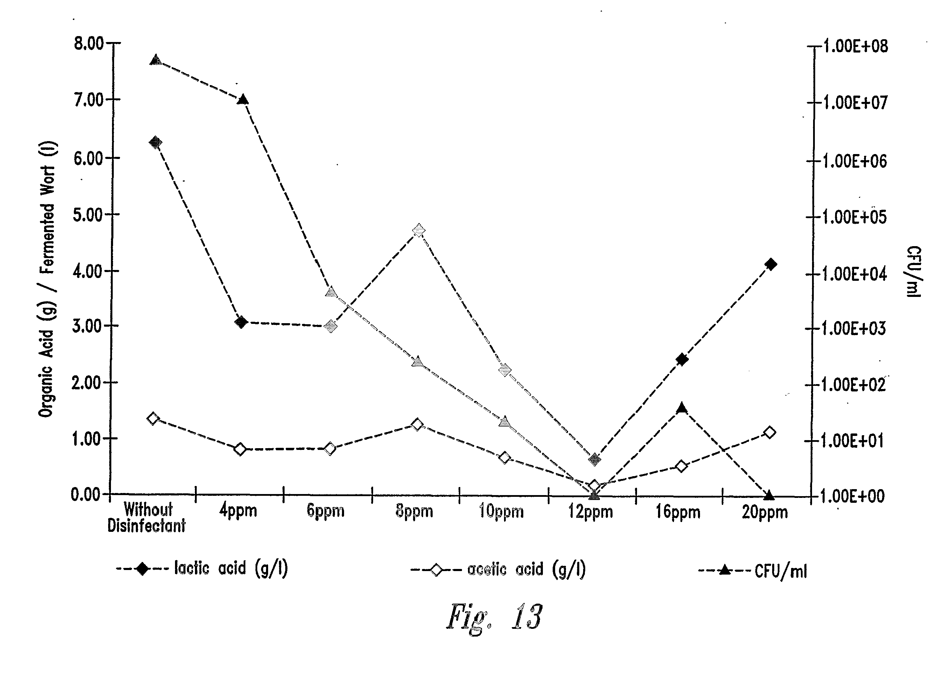

Figure 13 shows the decrease of bacterial metabolites produced by *Lb. brevis* at increasing concentrations of iso-a-acids in fermented molasses wort.

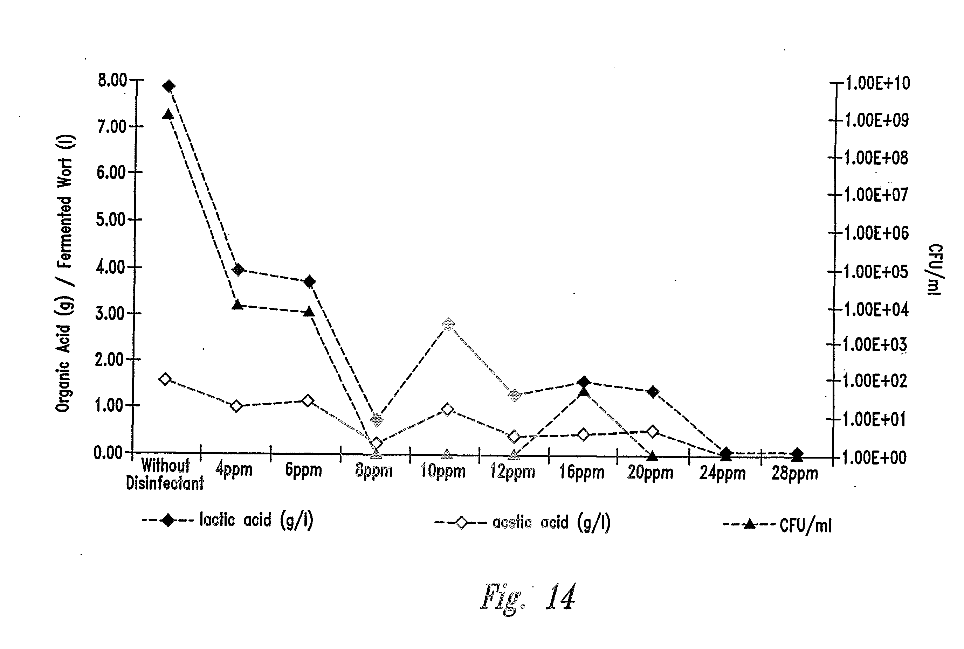

Figure 14 shows the decrease of bacterial metabolites produced by *Lb. fermentum* at increasing concentrations of iso-a-acids in fermented molasses wort.

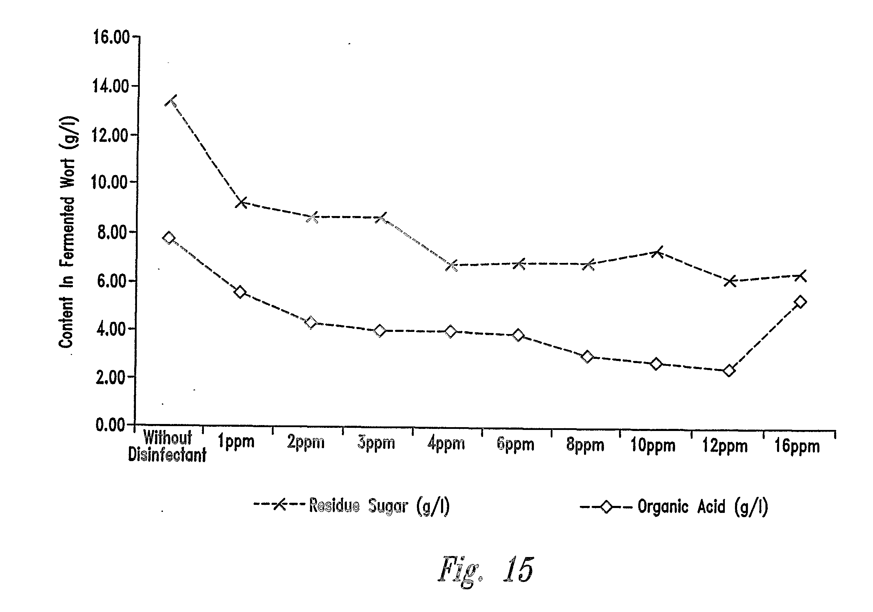

Figure 15 shows the synchronized decrease of bacterial metabolites produced by *Lb. brevis* and residue sugar at increasing concentrations of tetrahydro- iso- a- acids in fermented molasses wort.

Figure 15 shows the synchronized decrease of bacterial metabolites produced by *Lb. brevis* and residue sugar at increasing concentrations of hexahydro- iso- a- acids in fermented molasses wort.

Figure 17 shows the synchronized decrease of bacterial metabolites produced by *Lb. fermentum* and residue sugar at increasing concentrations of hexahydro- iso- a- acids in fermented molasses wort.

Figure 18 shows the synchronized decrease of bacterial metabolites produced by *Lb. brevis* and residue sugar at increasing concentrations of iso-a-acids in fermented molasses wort.

Figure 19 shows the synchronized decrease of bacterial metabolites produced by *Lb. fermentum* and residue sugar at increasing concentrations of iso-a-acids in fermented molasses wort.

Figure 20 shows the development of glucose- fructose- relation in residue sugar and ethanol yield at increasing concentrations tetrahydro- iso- a- acids in molasses wort. Molasses wort containing 129.74 g/L of sucrose was contaminated with initial bacterial cell numbers of $10^6$/mL *Lb. brevis.* Fermentation was carried out at pH 5.2 and 86°F for 96 hours.

Figure 21 shows the development of glucose- fructose- relation in residue sugar and ethanol yield at increasing concentrations tetrahydro- iso- a- acids in molasses wort. Molasses wort containing 129.74 g/L of sucrose was contaminated with initial bacterial cell numbers of $10^6$/mL *Lb. fermentum.* Fermentation was carried out at pH 5.2 and 96.8°F for 72 hours.

Figure 22 shows the development of glucose- fructose- relation in residue sugar and ethanol yield at increasing concentrations hexahydro- iso- a- acids in molasses wort. Molasses wort containing 129.74 g/L of sucrose was contaminated with initial bacterial cell numbers of $10^6$/mL *Lb. brevis.* Fermentation was carried out at pH 5.2 and 86°F for 96 hours.

Figure 23 shows the development of glucose- fructose- relation in residue sugar and ethanol yield at increasing concentrations hexadydro- iso- $\alpha$- acids in molasses wort. Molasses wort containing 129.74 g/L of sucrose was contaminated with initial bacterial cell numbers of $10^6$/mL *Lb. fermentum.* Fermentation was carried out at pH 5.2 and 96.8°F for 72 hours.

Figure 24 shows the development of glucose-fructose-relation in residue sugar and ethanol yield at increasing concentrations iso-a-acids in molasses wort. Molasses wort containing 129.74 g/L of sucrose was contaminated with initial bacterial cell numbers of $10^6$/mL *Lb. breves.* Fermentation was carried out at pH 5.2 and 86°F for 96 hours.

Figure 25 shows the development of glucose-fructose-relation in residue sugar and ethanol yield at increasing concentrations iso-a-acids in molasses wort. Molasses wort containing 129.74 g/L of sucrose was contaminated with initial bacterial cell numbers of $10^6$/mL *Lb. fermentum.* Fermentation was carried out at pH 5.2 and 96.8°F for 72 hours.

Figure 26 shows a comparison of ethanol yield. Molasses wort containing 129.74 g/L of sucrose was contaminated with initial bacterial cell numbers of $10^6$/mL *Lb. brevis.* Fermentation was carried out at pH 5.2 and 86°F for 96 hours.

Figure 27 shows a comparison of effectiveness in inhibition of *Lb. brevis.* Viable cell count by fast streak plate technique on MRS plates anaerobically incubated at 86°F for 48 hours.

Figure 28 shows a comparison of ethanol yield. Molasses wort containing 129.74 g/L of sucrose was contaminated with initial bacterial cell number of $10^6$/mL *Lb. fermentum.* Fermentation was carried out at pH 5.2 and 86°F for 96 hours.

Figure 29 shows a comparison of the effectiveness in inhibition of Lb. *fermentum.* Viable cell count by fast streak plate technique on MRS plates, anaerobic ally incubated at 96.8°F for 48 hours.

Figure 30 shows the development of ethanol yield at decreasing viable cell numbers of *Lb. brevis* correlated with increasing concentrations of tetrahydro- iso- $\alpha$- acids in wheat mash. Wheat mash containing 59.96 % of fermentable substance was contaminated with initial bacterial cell numbers of $10^7$/mL. Fermentation was carried out at pH 5.2 and 86°F for 96 hours.

Figure 31 shows the development of ethanol yield at decreasing viable cell numbers of *Lb. fermentum* correlated with increasing concentrations of tetrahydro- iso- $\alpha$- acids in wheat mash. Wheat mash containing 59.96 % of fermentable substance was contaminated with initial bacterial cell numbers of $10^7$/mL. Fermentation was carried out at pH 5.2 and 96.8°F for 72 hours.

Figure 32 shows the development of ethanol yield at decreasing viable cell numbers of *Lb. brevis* correlated with increasing concentrations of hexahydro- iso- $\alpha$- acids in wheat mash. Wheat mash containing 59.96 % of fermentable substance was contaminated with initial bacterial cell numbers of $10^7$/mL. Fermentation was carried out at pH 5.2 and 86°F for 96 hours.

Figure 33 shows the development of ethanol yield at decreasing viable cell numbers of *Lb. fermentum* correlated with increasing concentrations of hexahydro- iso- $\alpha$- acids in wheat mash. Wheat mash containing 59.96 % of fermentable substance was contaminated with initial bacterial cell numbers of $10^7$/mL. Fermentation was carried out at pH 5.2 and 96.8°F for 72 hours.

Figure 34 shows the development of ethanol yield at decreasing viable cell numbers of *Lb. brevis* correlated with increasing concentrations of iso-a-acids in wheat mash. Wheat mash containing 59.96 % of fermentable substance was contaminated with initial bacterial cell numbers of $10^7$/mL. Fermentation was carried out at pH 5.2 and 86°F for 96 hours.

Figure 35 shows the development of ethanol yield at decreasing viable cell numbers of *Lb. fermentum* correlated with increasing concentrations of iso-a-acids in wheat mash. Wheat mash containing 59.96 % of fermentable substance was contaminated with initial bacterial cell numbers of $10^7$/mL. Fermentation was carried out at pH 5.2 and 96.8°F for 72 hours.

Figure 36 shows the development of ethanol yield, content of residue sugar and bacteria metabolites at decreasing viable cell numbers of *Lb. brevis* correlated with increasing concentrations of tetrahydro- iso- a- acids in wheat mash.

Figure 37 shows the development of ethanol yield, content of residue sugar and bacteria metabolites at decreasing viable cell numbers of *Lb. fermentum* correlated with increasing concentrations of tetrahydro- iso- a- acids in wheat mash.

Figure 38 shows the development of ethanol yield, content of residue sugar and bacteria metabolites at decreasing viable cell numbers of *Lb. brevis* correlated with increasing concentrations of hexahydro- iso- a- acids in wheat mash. Figure 39 shows the development of ethanol yield, content of residue sugar and bacteria metabolites at decreasing viable cell numbers of *Lb. fermentum* correlated with increasing concentrations of tetrahydro- iso- a- acids in wheat mash.

Figure 40 shows the development of ethanol yield, content of residue sugar and bacteria metabolites at decreasing viable cell numbers of *Lb. brevis* correlated with increasing concentrations of iso-a-acids in wheat mash.

Figure 41 shows the development of ethanol yield, content of residue sugar and bacteria metabolites at decreasing viable cell numbers of *Lb. fermentum* correlated with increasing concentrations of iso-a-acids in wheat mash.

Figure 42 shows a comparison of ethanol yield. Wheat mash containing 59.9 % fermentable material was contaminated with initial bacterial cell numbers of $10^6$/mL Lb. *brevis.* Fermentation was carried out at pH 5.2 and 86°F for 96 hours.

Figure 43 shows a comparison of effectiveness in inhibition of *Lb. brevis* in wheat mash. Viable cell count by fast streak plate technique on MRS plates anaerobically incubated at 86°F for 48 hours.

Figure 44 shows a comparison of ethanol yield. Wheat mash containing 59.9 % fermentable material was contaminated with initial bacterial cell numbers of $10^7$/mL *Lb. fermentum.* Fermentation was carried out at pH 5.2 and 96.8°F for 72 hours.

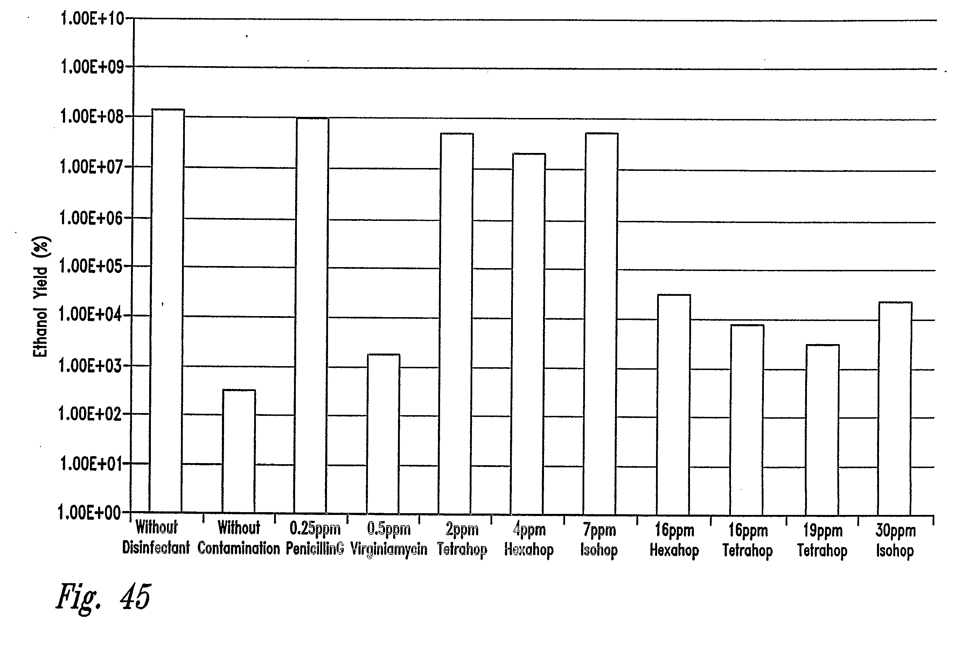

Figure 45 shows a comparison of effectiveness in inhibition of *Lb. fermentum* in wheat mash. Viable cell count by fast streak plate technique on MRS plates anaerobically incubated at 86°F for 48 hours.

Figure 46 is a diagram of the one embodiment of the process sequence for preparing an aqueous alkaline beta acid solution.

Figure 47 is a diagram of one embodiment for controlling the bacterial growth in a distillery where the fermentable solution is stored as a concentrate and the isomerized hop acid is dosed into the feed streams going to the yeast growing tanks and fermentors immediately after dilution.

Figure 48 is a diagram showing the dilution of concentrated molasses in the distillery treated in accordance with Example 7.

Figure 49 is a diagram demonstrating how the yeast in the yeast growing tanks were grown in the distillery treated in accordance with Example 7.

## DETAILED DESCRIPTION OF THE PREFERRED EMBODIMENT

The invention is directed to a process for controlling micro-organisms in an aqueous process medium comprising adding an aqueous alkaline solution of a hop acid to the process medium, wherein the pH of the aqueous alkaline hop solution is higher than the pH of the process medium.

The hop acid is a natural hop acid or a derivative thereof, such as, alpha acid, beta acid, tetrahydroalpha acid (THAA), or hexahydrobeta acid (HHBA), or mixtures thereof; an isomerized hop acid or a derivative thereof, such as, isoalpha acid (IAA), rhoiso alpha acid (RIAA), tetrahydro-isoalpha acid (THIAA) or hecahydro-isoallpha acid (HHIAA) or mixtures thereof. Alpha acids contained in the hop acid may be transformed into isoalpha acids during the preparation of the hop acid solution and maintain their anti-bacterial/anti-microbial effect.

Depending on the hop acid product, the concentration of hop acid in the aqueous solution will vary. For example, the concentration of THIAA in aqueous solution is generally 10 wt. % while the concentration of IAA can be as high as 30 wt. %. Generally, the final concentration of acid in the solution ranges from about 2 to about 40 wt. %, in another aspect from about 5 to about 20 wt. %, an in another aspect from about 10 to about 15 wt. %. Higher concentrations may be appropriate where longer transport times are required. Generally, hop acids in their acid form exhibit low solubility in water. However, hop acids can be mixed with an alkali metal hydroxide, for example potassium hydroxide, to make a water soluble alkali metal salt of the hop acid. According, it is advantageous to use alkali hydroxides, for example potassium hydroxide or sodium hydroxide or a mixture thereof as the alkaline medium to control micro-organisms. The concentrations of the alkaline medium ranges from about 20 % to about 45 wt. %, or in another aspect from about 20 wt. %.

As discussed above, the pH of the aqueous alkaline hop solution is higher than the pH of the process medium. As a result of the low dosage quantity of added solution compared to the process medium, the solution adapts almost entirely the pH of the process medium when added to the process medium and the hop acid passes from the salt form to the free acid, anti-bacterial effective, form. The pH of the aqueous alkaline hop acid solution added to the process medium ranges from about 7.5 to about 13.0, in another aspect from about 9.5 to about 11.0. A high bactericidal efficiency is achieved by using the solution in this range. The solution can be added without the danger of seriously damaging human skin. Furthermore, the solution does not create unpleasant or injurious vapors, unlike other chemical agents.

In one embodiment, the aqueous alkaline solution of hop acid is prepared according as follows:

a) provide an aqueous medium;
b) heat;

c) adding a hop acid, preferably, melted hop acid, such that the final concentration of the hop acid is within a predefined range of concentration;
d) adding an aqueous alkaline medium to obtain a pre-defined pH;
e) mixing the alkaline medium with the added hop acid;
f) maintaining the mixture in a raised temperature range within a predefined time period;
g) separating the solution of hop acid from the mixture and
h) cooling-down the solution of hop acid.

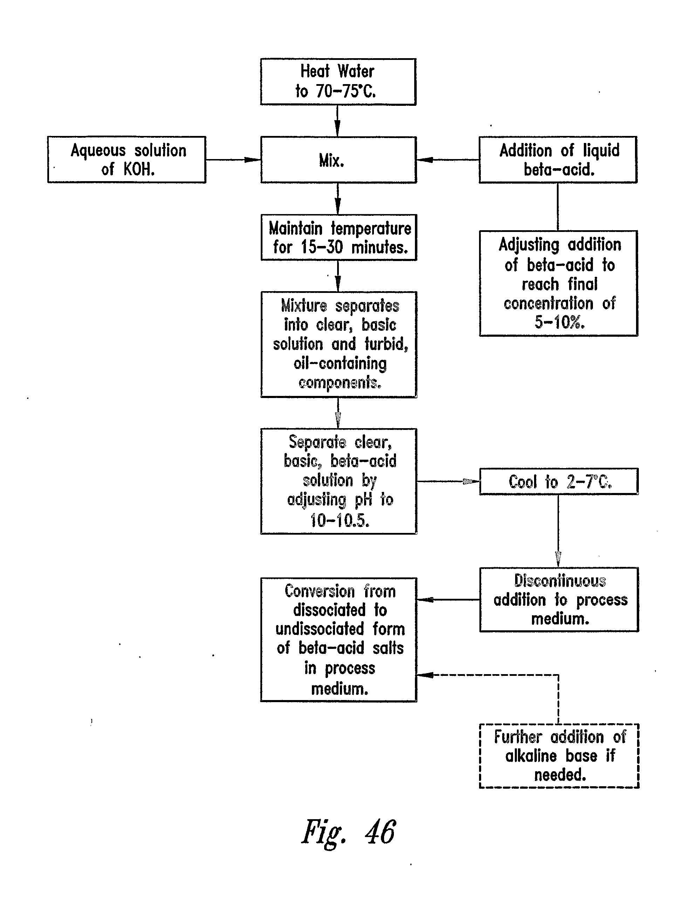

Figure 46 is a diagram of the process sequence for preparing an aqueous alkaline beta acid solution. In one embodiment, an aqueous solution of potassium hydroxide is heated from about 60 to about 80°C, in another aspect from about 65 to about 75°C, in yet another aspect from about 70 to about 75°C and the hop acid, e.g., melted beta acid, is added into to the potassium hydroxide solution. The temperature of the mixture is subsequently maintained for about 15 to 30 minutes or until the mixture separates into a clear, alkaline beta acid solution and an oil containing components. The clear, alkaline beta acid solution generally having a pH of about 10 to about 10.5 is separated from the mixture and is then cooled to a temperature below room temperature, such as to about 2 to about 7°C. This is subsequently dosed into the process medium discontinuously, e.g., by using shock dosage or continuously.

This process of preparing the aqueous alkaline solution of hop acid enables the preparation of a solution which can be stored and/or transported at higher concentrations of hop acids over longer periods. Under these conditions, these solutions are very stable. Its composition means that the solution can be dosed by pouring it in manually through hatches since it will not damage human skin, nor does the alkaline solution create unpleasant or injurious vapors unlike other chemical agents. Such solution provides appropriate characteristics for transport, the way to apply the solution and storage because of alkaline behavior. Also the pH of the solution is selected to ensure the highest possible increase in effect when it is used directly. The solution can also be dosed through the closed dosage systems for the emission free dosage of common anti-bacterial substances. The procedural steps are able to be changed in their sequence in time. The aforementioned sequence provides a very accurate definition of the pH of the aqueous alkaline hop acid solution.

In the process for controlling micro-organisms, the aqueous alkaline hop acid solution can be added to the process medium continuously or discontinuously, e.g., using shock dosage. For example, for shock dosage, the aqueous alkaline hop solution is periodically added to the process medium, e.g., the dosage is made at defined times within very short time intervals at which locally and for a short time interval high concentrations can be adapted. The high local concentrations achieved by this kind of dosing avoid the adaptation of the micro-organisms. The solution may be manually dosed into the process medium. Alternatively, the solution may be added to the process medium through closed dosing systems. That means that control of micro-organisms may be done under the use of the process installations (closed dosing systems) already available.

Generally, the temperature of the process medium to be treated is below 100°C, in one aspect below 50°C and in another aspect below 30°C. As discussed above, in the process medium the aqueous alkaline hop acid solution mixes with the slightly acid or at least less alkaline reacting process medium. As a result of the low dosage quantities of the highly concentrated hop acid solution, *e.g.,* beta acid or alpha acid solution, it adapts almost entirely to pH of the process medium, where upon the hop acid transforms from its salt form into the anti- bacterially and/or antimicrobially effective free acid form.

In another embodiment, melted, commercial hop acids, such as beta acids, can be directly added to the process medium. In such a process the melt is mixed with alkaline solution at an increased temperature shortly before a shock dosing. After the melt is dissolved, the entire mixture is dosed as a single shock. For short periods, strong alkaline conditions, which would lead to a loss of hop acids during interim storage, can be chosen.

The process for controlling micro-organisms can be automated by the use of time controls for the dosing pumps and valves. In this case, too, an increase of efficiency occurs. The improved effect means that the overall concentration of active ingredients can be reduced, which produces a number of advantages. Either reduced costs are achieved through lower dosing or the same dosing produces a better effect. For hop acids with the same concentration, the transport volume is reduced because of the greater efficiency.

The process for controlling micro-organisms can be applied in an advantageous way in distilleries for the production of non-beer alcoholic drinks, specifically of spirits or in the production process of wine and wine containing drinks, further in the production of natural ethanol, fuel ethanol, and pharmaceutical drugs. The process can also be used in the production of all kinds of dairy products, yeast, fruit juices and tinned foods in aqueous solution. Furthermore the process may be used in the formulation of cosmetic and detergent compositions.

It has also been discovered that isomerized hop acids and derivatives thereof are particularly effective at controlling the bacterial growth of distilleries. The isomerized hop acids are easier to use than traditional hops. Indeed, by using a standardized solution of isomerized hop acids, one is able to accurately dose the exact amount of hop acid required to control bacterial growth.

Accordingly, in another embodiment, a process for controlling the bacterial growth in a distillery is disclosed including adding an effective antibacterial amount of an isomerized hop acid to the process streams, e.g., yeast and/or fermentor streams of the distillery. In one embodiment, the process streams are treated with an alkaline aqueous solution of isomerized hop acid. Isomerized hop acids at concentrations as low as 2 ppm in the process medium can effectively control bacterial growth. Because isomerized hop acids are insoluble at concentration at about 100 ppm, localized high concentrations should be avoided.

Accordingly, the isomerized hop acid is preferably metered into the process very slowly, for example, by the use of small dosing pumps.

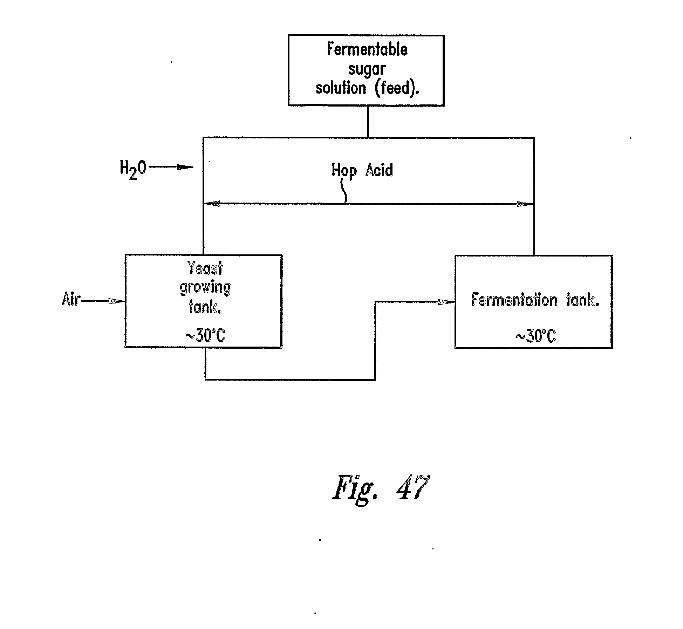

Figure 47 demonstrates an example where the fermentable solution is stored as a concentrate and the isomerized hop acid is dosed into the feed streams going to the yeast growing tanks and fermentors immediately after dilution. At very high concentrations, greater than 80 brix, no bacterial growth occurs, although the bacteria are still present in the feed material. After diluting the feed material to a fermentable concentration of about 25 brix, bacterial growth can occur. By adding the isomerized hop acid at this point in the process, bacterial growth can be inhibited right from the start.

An alternative to dosing the isomerized hop acid to both the yeast growing tanks as well as the fermentors is to dose a higher concentration of the hop acid just into the yeast growing tanks. Following yeast growth, the yeast solution containing the isomerized hop acid is transferred to an empty fermentor. As the fermentor is being filled, fermentation is taking place and the hop acid concentration is being diluted. If the correct amount of isomerized hop acid is added to the yeast growing tanks dilution in the fermentor will provide a final isomerized hop acid concentration of about 2 to about 4 ppm. At this concentration the isomerized hop acid can still control bacteria growth.

There are many advantages to using isomerized hop acids as antimicrobial agents for the distilling industry. First, hop acids are natural products which are used to bitter beer consumed by millions of people every day. Clearly, they are safe for human consumption. Further, because these hop acids have boiling points over 200°C, there is little need to be concerned with contaminating the distilled product with hops and therefore one can consider the use of hop acids as a processing aid. Finally, the dosing of isomerized hop acids is cost effective.

Hop acids are effective at controlling the growth of bacteria commonly found in fermentation streams. By controlling the growth of these bacteria, glucose can be converted into ethanol instead of lactic acid and acetic acid thus increasing ethanol yield. Although all hop acids reduced bacteria count, those which controlled the growth of microorganisms better because of solubility issues were THIAA, HHIAA and IAA. pH effects the minimum inhibitory concentrations (MIC) for hop acids. The lower the pH of the fermentation stream, the lower the amount of hop acids required to inhibit bacteria growth. Temperature also effects the antimicrobial properties of hop acids with the higher the temperature, the lower the MIC.

Generally, although a range of concentrations are possible, the MICs are about 2 ppm of TIAA, about 3 ppm of HHIAA or about 4 ppm of IAA to control bacteria growth in yeast propagators and fermenters. Because hop acids are insoluble at high concentrations and low pH's, in one aspect, hop acid concentration should be kept below 100 ppm hop acid. This can be accomplished through the use of metering pumps with a flow rate of 5- 30 liters per hour. By adding hop acids at the beginning of yeast growth and at the beginning of fermentation, bacteria growth can be inhibited from the start of the fermentation process.

Various concentrations of hop acids were tested in MRS broth, molasses wort, and w heat mash fermentations t o determine the minimum inhibitory concentration of the hop a cid toward *Lb. brevis* or *Lb. fermentum.* It was determined that hop acids inhibited the growth of bacteria in both the MRS broth and the fermentations, thereby increasing the percent of ethanol produced.

In MRS broth, various concentrations of alpha acids, beta acids, IAA, rho-isoalpha acids, THIAA, and HHIAA were added to MRS-broth treated with $10^6$ cells/mL of *Lb. brevis* or *Lb. fermentum.* In MRS-broth treated with $10^6$ cells/mL of *Lb. brevis,* pH 5.2, 30 °C, the treated broth was held for 60 hours to determine the MIC, as shown in Fig. 1. Although alpha acids and beta acids inhibited the growth of *Lb. brevis,* due to solubility issues, these acids were not further tested in fermentation experiments. The MIC of alpha acids assayed at about 14 ppm, beta acids about 10 ppm, rho-isoalpha acids about 20 ppm, isoalpha acid about 16 ppm, THIAA about 3 ppm and HHIAA about 3 ppm.

In another aspect, various concentrations of alpha acids, beta acids, isoalpha acids, rho-isoalpha acids, THIAA, and HHIAA were added to MRS-broth treated with $10^6$ cells/mL of *Lb. fermentum.* The MRS-broth, pH 5.2, 36 °C was held for 60 hours to determine the MIC as shown in Fig. 2. Although alpha acids and beta acids inhibited the growth of *Lb. fermentum,* due to solubility issues, these acids were not further tested in fermentation experiments. The MIC of alpha acids assayed at about 20 ppm, beta acids about 16 ppm, rho-isoalpha acids about 20 ppm, IAA about 8 ppm, THIAA about 2 ppm and HHIAA about 3 ppm.

MIC, minimum bactericidal concentration (MBC) and ethanol yields were also measured in molasses fermentations contaminated with $10^6$ cells/mL bacteria and treated with THIAA, HHIAA, and IAA as shown in Table 1. THIAA in molasses wort had a MIC of 3 ppm and MBC of 8 ppm for *Lb. brevis* and a MIC of 3 ppm and MBC of 6 ppm for *Lb. fermentum.* HHIAA in molasses wort had a MIC of 4 ppm and MBC 10 ppm for *Lb. brevis* and a MIC of 4 ppm and MBC of 8 ppm for *Lb. fermentum.* IAA in molasses wort had a MIC of 6 ppm and MBC of 12 ppm for *Lb. brevis* and a MIC of 4 ppm and MBC of 8 ppm for *Lb. fermentum.* The ethanol yield for each fermentation was compared to the control fermentation. Treating the fermentation streams with the MIC of the corresponding hop acids lead to on average a 10% increase in ethanol yield.

**Table 1. MIC, MBC and Ethanol Yield on Molasses Fermentations Treated with Hop Acids**

|  | *Lb. brevis* | *Lb. brevis* | *Lb. fermentum* | *Lb. fermentum* | % Ethanol (HPLC) | |
|---|---|---|---|---|---|---|
|  | MIC | MBC | MIC | MBC | *Lb. brevis* | *Lb. fermentum* |
| control | - | - | - | - | 86% | 80% |
| THIAA | 3 ppm | 8 ppm | 3 ppm | 6 ppm | 92% | 90% |
| HHIAA | 4 ppm | 10 ppm | 4 ppm | 8 ppm | 92% | 88% |
| IAA | 6 ppm | 12 ppm | 4 ppm | 8 ppm | 90% | 88% |

The molasses wort contained 129.7g/L sucrose, pH=5.2 and inoculated with $10^6$ bacteria cells/mL and held for 96 hours. The temperatures were 30 °C for *Lb. brevis* and 36 °C for *Lb. fermentum.* THIAA = tetrahydroisoalpha acids, HHIAA = hexahydroisoalpha acids, IAA = isoalpha acids.

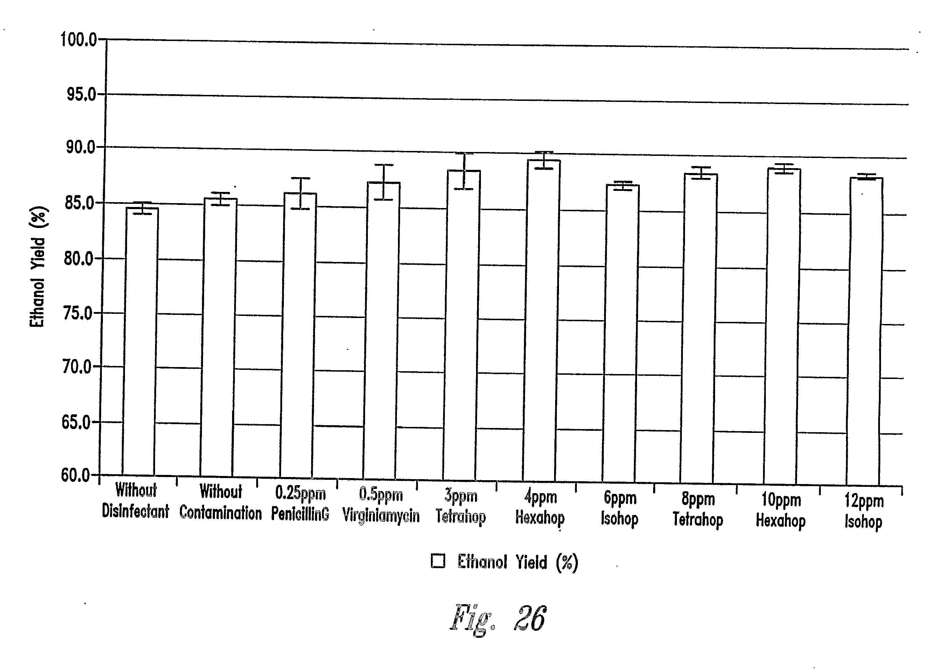

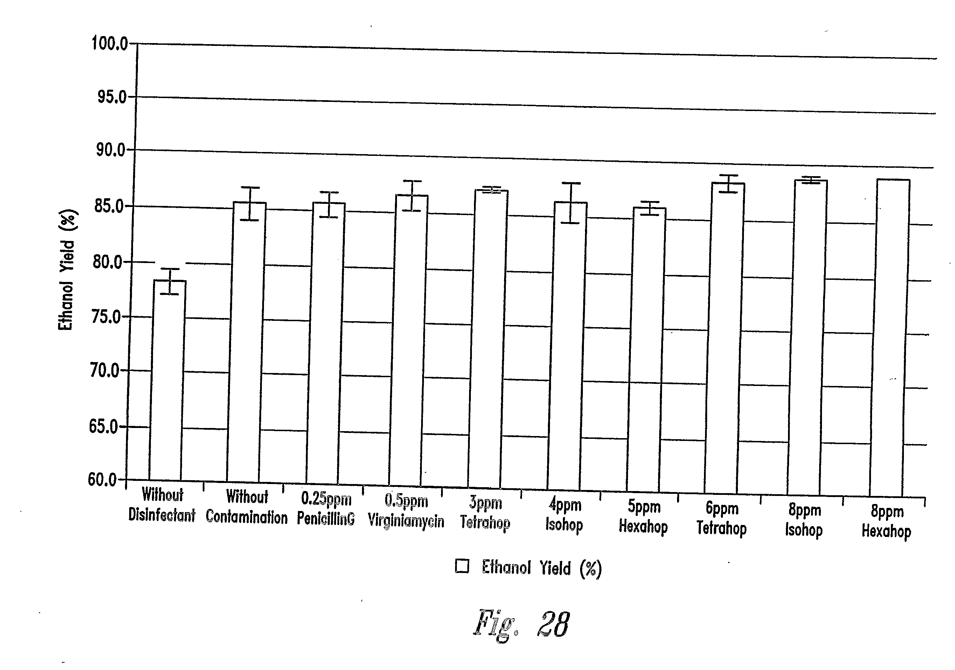

Figures 26 and 28 show that fermentations ran faster when hop acids were used instead of penicillin G and Virginiamycin.

MICs and ethanol yields were measured in wheat mash fermentations contaminated with $10^6$ cells/mL bacteria and treated with THIAA, HHIAA, and IAA as shown in Table 2. THIAA in wheat mash had a MIC of 6 ppm for *Lb. brevis* and a MIC of 4 ppm for *Lb. fermentum.* HHIAA in wheat mash had a MIC of 9 ppm for *Lb. brevis* and a MIC of 4 ppm for *Lb. fermentum.* IAA in wheat mash had a MIC of 14 ppm for *Lb. brevis* and a MIC of 9 ppm for *Lb. fermentum.* The ethanol yield for each fermentation was compared to the control fermentation. Treating the fermentation streams with the MIC of the corresponding hop acids resulted in an average 3-5% increase in ethanol yield.

**Table 2. MIC and Ethanol Yield on Wheat Mash Fermentations Treated with Hop Acids**

|  | *Lb. brevis* | *Lb. fermentum* | % Ethanol (HPLC) | |
|---|---|---|---|---|
|  | MIC | MIC | *Lb. brevis* | *Lb. fermentu* m |
| control | - | - | 86% | 90% |
| THIAA | 6 ppm | 4 ppm | 90% | 94% |
| HHIAA | 9 ppm | 4 ppm | 88% | 93% |
| IAA | 14 ppm | 9 ppm | 90% | 92% |

The wheat mash contained 15.7% solids, 60% fermentable substance, pH=5.2 and inoculated with $10^7$ bacteria cells/mL and held for 96 hours. The temperatures were 30 °C for *Lb. brevis* and 36 °C for *Lb. fermentum. .* THIAA = tetrahydroisoalpha acids, HHIAA = hexahydroisoalpha acids, IAA = isoalpha acids.

In the fermentation experiments discussed below with sugar beet molasses wort as medium, lactic acid bacteria were inoculated directly in used up MRS- broth. This technique was responsible for high initial concentrations of lactic acid and acetic acid in the wort and helped to visualize the effect of lactic acid bacteria contamination of worts by losses in ethanol yield. Even when bacteria are present in high numbers in yeast- mediated fermentations, they must create biomass quickly in order to create enough metabolic potential to compete with yeast cells for sugar and create ethanol yield reducing levels of lactic acid prior to termination of fermentation (Narendranath, N.V., et al, Appl. & Envir. Microbiol., 63 (11) : 4158- 4163, 1997) . The specification of the amount of organic acids in the following refers to the amount of organic acids (e.g. lactic acid and acetic acid) produced during fermentation.

The decrease in viable cell numbers of lactic acid bacteria at increasing concentrations of hop acids went along with a measurable decrease of bacteria metabolites in fermented sugar beet molasses wort. In worts fermented with an undamped contamination of lactic acid bacteria, the content of lactic acid and acetic acid produced by the bacteria during fermentation was approximately three times as high as in worts in which the bacteria had been successfully inhibited.

Parallel to the decrease of organic acids, the consumption of sugars by yeast was improved and the content of residue sugar, consisting of raffinose, sucrose, glucose and fructose, in the fermented wort decreased. The glucose-fructose relation in total residue sugar improved, while the unused portion of raffinose and sucrose was small and remained constant. The consumption of sugar by yeast is dependent on the glucose-fructose-relation in the medium. A glucose-fructose relation less than 0.2 restricts yeast activity. Where growth of lactic acid bacteria was undampened, glucose was usually totally consumed by yeast and bacteria and high contents of fructose remained, provoking losses in ethanol yield up to about 15 %. In worts, in which the growth of lactic acid bacteria had been successfully suppressed, residue sugar contained glucose and fructose in a 1:2 relationship. Further, ethanol yields improved to about 90 % and above.

Yeast growth is affected when the bacterial concentration exceeds 104 CFU/mL (Essia, N et al., Appl. Microbiol. Biotechnol.; 33: 490 -493, 1990.) In accordance with this, best ethanol yields were achieved when the viable number of bacteria was reduced below 104 CFU/mL and could generally not be improved any further by continued reduction of bacterial cells at higher concentrations of hop acids. The specific hop acid concentration at which bacterial numbers are reduced below $10^4$/mL is the "effective concentration".

1. <u>Materials and Methods</u>

In conducting the experiments described in the Example 1-5, the following materials and methods were used. Variations known to one of skill in the art in the materials and methods are encompassed herein.

Bacteria used

Two species of the genus Lactobacillus, both isolated from sourdough, were used: *Lactobacillus brevis* (LTH 5290) and *Lb. fermentum* (LTH 5289). Preliminary tests showed that both species were capable of growth in sugar beet molasses wort as well as in wheat mash and were tolerant to more than 9% (vol/vol) ethanol. Bacterial count in stationary phase cultures which had been bred in, respectively, sugar beet molasses wort and wheat mash did not differ from bacterial count in stationary phase cultures bred in de Man-Rogosa-Sharpe (MRS) broth. ($10^7$-$10^8$ CFU/mL) Both strains belong to the family of heterofermentative lactobacilli, are able to ferment sucrose and their glucose-metabolism produces one mole lactic acid (DL-form), one mole acetic acid and ethanol, and one mole $CO_2$ per mole glucose. The optimal temperature for growth is 86°F of for *Lb. brevis* and 98.6°F of for *Lb. fermentum.* Fermentation essays were at each case carried out at the appropriate optimum temperature for the contaminant. Fermentation time was adapted to total consumption of sugar by yeast in an undisturbed fermentation at each temperature condition. Worts contaminated with Lb brevis were incubated for 96 hours at 86 °F; worts contaminated with Lb. fermentum were incubated for 72 hours at 98.6 °F.

Media

De Man Rogosa Sharp Medium (Fa. Merck, Darmstadt) was used for maintenance of the test organism. After having noticed that the bacteria would not grow well, as some of the glucose was made unavailable in Maillard reactions during autoclaving, the medium was enriched with sterile glucose-solution after sterilization, adding 5 g/L of glucose to MRS-broth and MRS-agar. This medium is referred to as MRS.
For estimation of MIC, the pH value of the medium was adjusted to pH 5.2 with concentrated HCl before sterilization. This modified medium is referred to as modified MRS.

(i) Preparation of bacterial inocula for sugar beet molasses wort

The clean breed strains were kept frozen at -101.2°F in MRS-broth containing 8%-glycerol and were inoculated from there in 10 mL cap tubes containing 2 mL MRS-broth. The headspace of each tube was flushed with filter sterilized (0.45 $\mu$m pore size membrane filter) $CO_2$-gas and the caps were sealed with paraffin wax coated film. The tubes were incubated in a controlled environmental shaker at 100 rpm at 86°F (Lb. *brevis*) respectively 96.8°F (*Lb. fermentum*). After 12 hours, 1 mL of these preparatory cultures were each transferred into 10 mL cap tubes containing 9 mL MRS-broth and  incubated for another 24 hours, afterwards transferred to 100 mL screw cap flasks containing 90 mL of MRS-broth and again incubated at the appropriate temperature for 24 hours. After that the bacterial cells were aseptically harvested in sterile centrifugal tubes by centrifugation at 10,200x g for 15 minutes at 4 °C. The pellets were washed twice with sterile 1 % peptone water and resuspended in 20 mL of sterile 0.85 % saline solution. These portions were transferred to 1 L screw cap flasks, containing 750 mL MRS-broth and were again incubated for 24 hours. Cell numbers of the organisms were estimated using a Beck photometer. An even function describing the relationship between the optical density against MRS-broth at 578 nm wavelength and the number of colony forming units per mL was established for both strains. The inoculation of sugar beet molasses wort with lactobacilli took place directly in MRS-medium instead of adding yeast extract as nutrient supplement for yeast. A filter sterilized (0.45 $\mu$m pore size membrane filter) 5 $\mu$l aliquot of the MRS-cell suspension for inoculation was determined by high performance liquid chromatography using a ProntoSIL 120-3-C18 AQ column which analyzes sugars, organic acids and alcohol, making sure glucose in the MRS-medium would be totally consumed and determining the amount of lactic acid an acetic acid added to fresh wort. Appropriate quantities of cell suspension were added to give a total of 500 g mash in laboratory fermentation flasks and initial viable bacterial cell numbers of $10^6$ CFU/mL mash. The pH-value of the wort was afterwards readjusted to pH 5.2 if necessary.

(ii) Preparation of bacterial inocula for wheat mash

The clean breed strains were kept frozen at -101.2°F in MRS-broth containing 8 %-glycerol and were inoculated from there in 10 mL cap tubes containing 2 mL MRS-broth. The headspace of each tube was flushed with filter sterilized (0.45 $\mu$m pore size membrane filter) $CO_2$-gas and the caps were sealed with paraffin wax coated film. The tubes were incubated in a controlled environmental shaker at 100 rpm at 86 °F (*Lb. brevis*) and 96.8 °F (*Lb. fermentum*). After 12 hours 1 mL of these preparatory cultures were each transferred into 10 mL cap tubes containing 9 mL MRS-broth and incubated for another 24 hours, afterwards transferred to 100 mL screw cap flasks containing 90 mL of MRS-broth and again incubated at the appropriate temperature for 24 hours. These portions were transferred to 1 L screw cap flasks, containing 750 mL MRS-broth and were again incubated for 24 hours.

For inoculation of wheat mash the bacterial cells were aseptically harvested in sterile centrifugal tubes by centrifugation at 10,200 x g for 15 minutes at 4 °C. The  pellets were washed twice with sterile 1 % peptone water and resuspended in 20 mL of sterile 0.85 % saline solution. Such harvested bacterial cells of each strain were reunited to give a concentrated cell suspension and were kept at 39.2 °F until they were dispensed.

Cell numbers of the organisms were estimated using a Beck photometer. An even function describing the relationship between the optical density at 578 nm wavelengths against 0.85 % saline solution and the number of colony forming units per mL was established for both strains. Appropriate quantities of the concentrated cell suspension were added to 500 g quantities of wheat mash in laboratory fermentation flasks to give initial viable cell numbers of $10^7$ CFU/mL.

Preparation of yeast inoculum

The number of viable cells per gram of S. *cerevisiae* active dry yeast (Schlienzmann Brennereihefe forte) was determined by enumeration of yeast cells on YPD medium. 0.1 g, 0.5 g and 1 g of S. *cerevisiae* active dry yeast were dispensed into 10 mL of sterile 0.85 % saline solution and incubated at 86°F for 30 minutes. A dilution series from $10^{-1}$ to $10^{-9}$ was made of each suspension and viable cell count was determined by streak plate technique. Viable cell counts were multiplied with factor 10 to eliminate the initial dilution by calculation. Enumeration resulted in approximately $10^9$ viable yeast cells per gram active dry yeast.

Fermentation time was monitored subject to osmotic pressure and content of sugars in the wort, fermentation temperature and yeast dosage in order to minimize the initial viable cell number of yeast. This was necessary to achieve visible ethanol losses in laboratory scale fermentations. As has been reported by Hynes S.H. et al. (J. Indust. Microbio. and Biotech. 18 (4): 284-291, 1997) (and various other authors), even undamped growth and lactic acid production by bacteria is often not sufficient to have an effect on fermentation if the yeast inoculum in the mash is high ($10^7$ yeast/g mash). In the tests described in the examples below, a yeast inoculum of 0.6 g active dry yeast for 500 g wort was used, which corresponds to an initial viable cell number of 1.2 x $10^6$. The effects might have been even bigger with smaller yeast numbers but this inoculum was necessary to complete undisturbed fermentation in sugar beet molasses containing 130 g/L sucrose within 72 hours, as desired.

For each fermentation sample of 500 g wort, 0.6 g of S. *cerevisiae* active dry yeast was dispersed into 10 mL of tap water and incubated at 86 °F for 30 minutes. After manual shaking, the suspension was added to the laboratory fermentation flask.

Preparation of inhibitory substances

(iii) Preparation of hop extracts

Six differently composed $CO_2$ hop extracts available from Haas Hop Products, Inc., Washington, D.C., were tested for both Lactobacillus strains. The Haas Hop Products tested were: (1) Alphahop®, a pure standardized highly concentrated resin composition of 92% $\alpha$- acids; (2) Betastab®, a pure standardized composition of 10% $\beta$- acids and essential hop oils; (3) Redihop®, a pure, standardized solution of 35 % rhoiso- $\alpha$- acids; (4) Isohop, a pure standardized solution of 30 % iso- $\alpha$- acids; (5) Hexahop Gold™ a pure standardized solution of greater than 8% hexahydro- iso- a-acids and (6) Tetrahop™, a pure standardized solution of 10% tetrahydo- iso- $\alpha$- acids. The differently concentrated $CO_2$ hop extracts were diluted in deionized sterile water in a manner that all dilutions contained 0.001 % hop acids. Alphahop® was dissolved 1: 1 in 95% ethanol before diluting because of its poor solubility in water.

Generally, hop acids exhibit low solubility in water. However, hop acids can be mixed with an alkali metal hydroxide, preferably potassium hydroxide, to make a water soluble alkali metal salt of the hop acid. Accordingly, in the process for controlling micro- organisms, it is advantageous to use alkali hydroxides, specifically potassium hydroxide or sodium hydroxide or a mixture thereof, as the alkaline medium. The concentrations of the alkaline medium preferably ranges from about 1 to about 4. wt. %, more preferably from about 2 to about 3 wt. %.

As discussed above, in the method described herein for lowering the concentration of lactic acid producing bacteria, the pH of the aqueous alkaline hop solution added to the process medium is higher than the pH of the process medium. As a result of the low dosage quantity of added solution compared to the process medium, the solution adapts almost entirely the pH of the process medium when added to the process medium and the hop acid passes from the disassociated form (salt form) to the associated (free acid), anti-bacterial effective, form. In one aspect, the pH of the aqueous alkaline hop acid solution added to the process medium ranges from about 7.5 to about 13.0, in another aspect from about 9.5 to about 11.0. A high bactericidal efficiency is achieved by using the solution in this range. The solution can be added without the danger of seriously damaging human skin. Furthermore, the solution does not create unpleasant or injurious vapors, unlike other chemical agents.

Preliminary testing of the MIC showed that Isohop®, Hexahop Gold™ and Tetrahop™, because of solubility issues, were the most effective against bacteria. These three products were used for testing the potency as a disinfectant in molasses wort and wheat mash. Appropriate quantities of the dilutions described above were added to mash to give concentrations in a range from 1 to 28 ppm of prepared mash.

(iv) Preparation of Virginiamycin

Stafak® containing 10 % Virginiamycin was the source of Virginiamycin. Hynes S.H. et al. (J. Indust. Microbio. and Biotech. 18 (4): 284-291, 1997) reported a concentration of 0.5 mg Virginiamycin per kg mash is effective against most of lactic acid bacteria. 0.125g Stafak® was dissolved in 50 mL deionized sterile water to obtain a dilution containing 0.25 mg Virginiamycin per mL. One milliliter of this dilution was added to 500 g wort to give a concentration of 0.5 ppm in the wort.

(v) Preparation of Penicillin G

Penicillin G Sodium for technical use in distilleries, available from Novo Industri A/S, Denmark, was used according to manufacturer's instructions of 1 g Penicillin G as sufficient for 4000/wort. 12.5 mg Penicillin G was dissolved in 100 mL deionized sterile water to obtain a dilution containing 0.125 mg/mL. .1 mL of this dilution was added to 500 g wort to give a concentration of 0.25 ppm in the wort.

(vi) Preparation of molasses wort and fermentation

The content of sucrose in beet molasses was determined by polarimeter after clarification with lead acetate. Beet molasses, about 78 % dry matter and about 49.9 % sucrose (w/w), were diluted with distilled water to obtain worts containing 129.74 g/L of sucrose. The wort was heated to 176 °F, adjusted to pH 5.2 with 1 N $H_2SO_4$ and stirred at 176° F for 30 minutes in order to pasteurize the wort and to invert a great part of sucrose to glucose and fructose. Preliminary testing of the biological fermentation qualities showed that it would not be necessary to defoam or to filtrate the wort.

After that the mash was cooled to 86 °F for *Lb. brevis* and 98.6 °F for *Lb. fermentum.* At this point, various concentrations of hop extracts diluted in deionized sterile water or conventional antibiotics diluted in deionized sterile water were added to the wort. Just prior to yeast inoculation, the samples were contaminated with bacteria to give initial viable cell numbers of $10^7$ CFU/mL and afterwards transferred quantitatively to 1 L fermentation flasks, filled up with tap water to 500 g and closed with rubber stoppers with fermentation tubes.

Further tests showed that sterilized MRS-broth which had been used up by Lactobacillus breed could replace yeast-extract solution as yeast nutrient supplement. In the following experiments described below, Lactobacilli were directly added in used up MRS -Medium containing no sugars, an aliquot of sterilized used up MRS-broth was added to contamination free samples.

Fermentations were carried out at 86 °F for 96 hours when inoculated with *Lb*. *brevis* and at 98.6 °F of for 72 hours when inoculated with *Lb. fermentum* in 1 L laboratory fermentation flasks containing 500 g wort.

(vii) Mashing of wheat and fermentation (a) Determination of fermentable substance

Commercial winter wheat was ground at a 0.5 mm setting on a Retsch model SR2 Haan disk mill, available from Retsch GMBH & Company, Germany. The amount of fermentable substance, such as maltose, glucose and fructose, was analyzed by HPLC method (Senn 1988)..10 g of ground wheat +/- 0.001 g was dispensed in 300 mL tap water. The pH value was adjusted to pH 6.0 - 6.5 with 1 N NaOH, then 0.2 mL of high temperature α-amylase (Optimash pH 420, Solvay Enzymes, Hanover) was added to create a probe. The probes were heated to 203 °F in a model MA-3E VLB-mash bath (Bender and Hohbein, Munich) and kept at this temperature for 60 minutes. Then the temperature was cooled to 131 °F, the pH-value was adjusted to pH 5.0 -5.3 with 1 N $H_2SO_4$ and saccharification enzymes were added (0.2 mL Fungal-$\alpha$-amylase L40000, available from Solvay Enzymes, Hanover; 2 mL SAN Super 240L, available from Novo, Bagsvaerd, Denmark; 0.1 mL Optilase F300, available from Solvay Enzymes, Hanover). Saccharification took place overnight. Afterwards the probes were cooled to 68 °F, transferred quantitatively to 1 L graduated flasks, filled up with distilled water to the 1 L marking and first filtered by a wave filter, then membrane filtered by a 0.45 $\mu$m pore size filter. A 10 $\mu$l aliquot of the filtrate was analyzed by HPLC using a ProntoSIL 120-3-C18 AQ column which analyzes sugars, organic acids and alcohol to determine the content g/L of maltose, glucose and fructose. For determination of blank values, 250 mL tap of water with enzymes but without ground wheat were used. The amount of fermentable substance was calculated after subtracting blank values:

$$[(((\text{Glucose [g/L]} + \text{Fructose [g/L]]} \times 0.899) + (\text{Maltose [g/L} \times 0.947)) / \text{ground wheat dosage}] \times 100$$

(b) Standard laboratory process for mashing and fermentation of wheat

Commercial winter wheat was ground at a 0.5 mm setting on a Retsch model SR2 Haan disk mill. For mashing, 80 g ground wheat per sample (59.96 % fermentable substance (w/w)) was dispensed in 300 mL tap water. The samples were placed in a model MA-3/E mash bath (Bender & Hohbein, Munich) and high temperature bacterial $\alpha$-amylase was added. The temperature was raised to 149 °F to gelatinize the starch. The mash was held for 30 minutes at this temperature to complete liquefaction. The preparation was then cooled to a 125.6 ° F saccharification temperature and held at that temperature for another 30 minutes. The pH value was adjusted to pH 5.2 with 1 N $H_2SO_4$. Saccharification of dextrin to glucose was carried out by adding 0.625 mL of glucoamylase (SAN Super 240 L of *Aspergillus niger,* (Novo, Bagsvaerd, Denmark) per sample. After that the mash was cooled to 86 °F for *Lb. brevis* and 98.6 °F for *Lb. fermentum.* At that point, various concentrations of hop extracts diluted in sterile deionized water or conventional antibiotics diluted in sterile deionized water were added to the wort. Just prior to yeast inoculation, the samples were contaminated with bacteria to give initial viable cell numbers of $10^7$ CFU/ mL and afterwards transferred quantitatively to 1 L fermentation flasks, filled up with tap water to 500 g and closed with rubber stoppers with fermentation tubes. Fermentations were carried out at 86 °F for 96 hours when inoculated with *Lb. brevis* or at 98.6 °F for 72 hours when inoculated with *Lb. fermentum* in 1 L laboratory fermentation flasks containing 500 g wort.

(viii) Assay methods

(a) Assay of minimum inhibitory concentration (MIC)

The MICs of $\alpha$- acids, $\beta$- acids, iso- $\alpha$- acids, rho- iso- $\alpha$- acids, hexahydro- iso- a- acids and tetrahydo- iso- $\alpha$-acids were determined by tube dilution technique. All tests were performed at least twice with independently prepared media and test solutions. The test inoculum was prepared by aseptically harvesting bacterial cells of a mid- log- phase culture in MRS broth by centrifugation at 10, 200 x g for 15 minutes at 4 °C. The pellets were washed twice with sterile 1 % peptone water and resuspended in 20 mL of sterile 0.85 % saline solution. Such harvested bacterial cells of each strain were reunited to give a concentrated cell suspension and were kept at 39.2 °F until they were dispensed. After determining cell numbers by measuring the optical density with a Beck photometer, appropriate quantities of concentrated cell suspension were added to 10 mL modified MRS- broth, containing a range of hop compounds and hop derived compounds, to give initial viable cell numbers of $10^6$/mL and $10^7$/mL. The tubes were incubated anaerobically in anaerobic jars with Anaerocult® A (available from Merck, Darmstadt) at 86 °F for *Lb. brevis* and 98.6 °F for *Lb. fermentum* for 60 hours. Growth was assessed photometrically at 578 nm against modified MRS- broth in disposable plastic microcuvettes in a Beck photometer.

(b) Determination of ethanol yield in fermented wort

The distillation was carried out with programmable water vapor distillation equipment with probe distillation model Vapodest (available from Gerhardt, Bonn). 50 g of wort was transferred into a distillation flask. 0.25 N NaOH was immediately added to adjust pH to 7.0 to keep organic acids from being carried over, and after a reaction time of 2 seconds water vapor distillation was started at 85 % performance for 225 seconds. The distillate was caught in a 100 mL graduated flask, topped up to the 100 mL marking with deionized water, and set at a temperature of 68 °F.

For determination of ethanol yield, a digital density meter model DMA 48 (available from Chempro, Hanau) was used. A defined volume of distillate was introduced in the density meter's u-shaped sampling tube. This sampling tube has a bearing, which is able to oscillate. Undamped oscillation is stimulated by the increased mass of the tube. At constant temperature, the introduced mass is commensurate to the density. The cycle duration of the oscillating system is the computation base for the density. The reference temperature is 68 °F. The density values were translated to percent by volume with the aid of table 6 of *Amtliche Alkoholtafeln'* and multiplied by a factor of 2 to account for the dilution of the 50 g wort sample in 100 mL distillate. The ethanol yield of 100 kg raw material is calculated as follows:

$$[l\ A\ /\ dt\ raw\ material] = \frac{alcoholic\ content\ of\ the\ distillate\ [vol/vol]\ x\ weight\ of\ fermented\ mash\ [g])}{initial\ weight\ of\ raw\ material\ [g]}$$

The ethanol yield of 100 kg fermentable material is calculated as follows:

$$[l\ A\ /\ dt\ term\ material] = [l\ A\ /\ dt\ raw\ material]\ x\ 100]\ /\ fermentable\ material\ [\%]$$

(c) Viable counts of bacteria cells

Viable cell counts were monitored by a rapid method of streak plate technique (Baumgart, J.: Mikrobiologische Untersuchungen von Lebensmitteln, Behr's Verlag, Hamburg, 1994). MRS-plates were subdivided into six similar pieces, like in a pie chart. From each sample of fermented wort a dilution series from 10 to $10^{-6}$ was made in sterile saline solution and a 50 $\mu$l drop of each dilution was carefully set up on the surface of one piece of the six pieces. Twelve plates at a time were incubated anaerobically in an anaerobic jar with Anaerocult® A (available from Merck, Darmstadt) and incubated for 48 hours at the appropriate temperature (86 °F for *Lb. brevis* contamination, 98.6 °F. *for Lb. Fermentum* contamination). Pieces containing between 5 and 50 colonies were taken for enumeration. The number of colony forming units per mL wort was calculated as weighted average:

$$CFU/\ mL = [\Sigma\ C\ /\ (n_1\ x\ 1 + n_2\ x\ 0.1)]\ x\ d$$

$$\Sigma\ C = number\ of\ colonies\ at\ lowest\ numerable\ dilution + number\ of\ colonies\ at\ highest\ numerable\ dilution$$

$n_1$ = number of plates at lowest numerable dilution
$n_2$ = number of plates at highest numerable dilution
d = 1/lowest numerable dilution

(d) HPLC analysis

Residue sugars (raffinose, sucrose, maltose, glucose, fructose), organic acids (lactic acid, acetic acid) and ethanol in the fermented wort were determined by HPLC analysis using a ProntoSIL 120-3-C18 AQ column maintained at 122 °F after calibration with standards of analytical grade. A filter sterilized (0.45 $\mu$m pore size membrane filter) 5 $\mu$l aliquot of the mash was injected. The determination was done in duplicate for each sample. 0.01 N $H_2SO_4$ was used as the mobile phase at flow rate of 0.6 mL/minute. The components were detected with a differential refracting index detector RI 16. The data were processed by Bischoff McDAq Software.

(e) Provoking resistances and monitoring cross resistances

Survivors of *Lb. brevis* and *Lb. fermentum* were isolated from viable cell count plates out of molasses worts with the highest concentration of iso-$\alpha$- acids, hexahydro- iso- a- acids and tetrahydo- iso- $\alpha$- acids, which had allowed some few organisms to survive. These colonies were transferred from MRS- plates into 10 mL modified MRS- broth, containing a moderate concentration of the special hop compound, the organism had survived. The headspace of each tube was flushed with filter sterilized (0.45 $\mu$m pore size membrane filter) $CO_2$- gas, the caps were sealed with paraffin wax coated film and incubated in a controlled environmental shaker at the appropriate temperature for the particular bacteria for 48 hours. Control tubes contained no hop acids at all. Afterwards 100 μl of each sample was spread on the surface of MRS- plates using streak plate technique and incubated anaerobically in anaerobic jars with Anaerocult® A at the appropriate temperature for 48 hours for regeneration. The plating was done in duplicate for each sample. This process was repeated ten times, each time the concentration of the monitored hop compound in the tubes was raised 1 ppm.

Out of this series, only *Lb. brevis* colonies survived. They were transferred into 10 mL modified MRS- broth, containing a range of the two other hop compounds in order to test cross resistances. The tubes were treated as described above.

2. Examples

Using the above described materials and methods and their variations, various tests were performed to find the inhibitory concentration of hop acids, including tests to determine the minimum inhibitory concentrations and the effective concentrations of hop acids which can be used to reduce or eliminate lactic acid and/or acetic acid producing bacteria during the production of fuel ethanol and spirits. The following Examples are intended to illustrate, but not limit, the scope of this invention.

**Example 1: The determination of the MIC**

Alphahop®, a pure standardized highly concentrated resin composition of 92% α- acids; Betastab®, a pure standardized composition of 10% β- acids and essential hop oils; Redihop®, a pure, standardized solution of 35% rho- iso- α- acids; Isohop®, a pure standardized solution of 30 % iso- α- acids; Hexahop Gold™, a pure standardized solution of about 8 % or greater than 8 % hexahydro- iso- α- acids and Tetrahop™, a pure standardized solution of 10 % tetrahydo- iso- α- acids, all available from John I Haas, Inc. Haas Hop Products or Washington, D.C., USA, were tested to determine the concentration which would have an effect to reduce and/or eliminate acetic acid and/or lactic acid producing bacteria. Specifically used in the test were *Lb. brevis* and *Lb. fermentum,* although other types of bacteria may also be controlled.

As shown in Figures 1 and 2, Alphahop®, Betastab® and Redihop® inhibited growth compared with control tubes containing no hop compound (100% growth), but had, due to their poor solubility in water, only weak antibacterial effect compared to Isohop®, Hexahop Gold™ and Tetrahop™. The minimum inhibitory concentrations (MICs), the concentrations at which some control of microorganism is seen, for Alphahop®, Betastab® and Redihop® range around 20 ppm or higher. Therefore, only Isohop®, Hexahop Gold™ and Tetrahop™ went into the fermentation tests.

As shown in Figures 1 and 2, *Lb. fermentum* proved to be more sensitive to the ionophoric action of hop acids than *Lb. brevis.* The MIC of Isohop® for *Lb. brevis* was about 16 ppm and for 8 ppm for *Lb. fermentum.* HHIAA proved to have excellent antibacterial properties with an MIC of between 3-6 ppm for both strains and THIAA came out on top with an MIC of 3 ppm for *Lb. brevis* and 2 ppm for *Lb. fermentum.*

**Example 2: Determination of Effective Concentration and Optimum concentration of Hop Acid**

The effective concentrations required for THIAA, HHIAA and IAA did not differ much between *Lb. brevis* and *Lb. fermentum. Lb. fermentum* was more sensitive and at increased concentration all bacteria were killed, while numbers of *Lb. fermentum* could only be extensively reduced to a dimension of approximately $10^1$-$10^2$ mL. The concentration at which bacterial numbers are minimal or eliminated is the "optimum concentration".

As shown in Figure 3, the effective concentration of THIAA for the inhibition of *Lb. brevis* was about 3 ppm. The optimum concentration at which viable cell numbers were extensively reduced was about 8 ppm. There was no improvement in reduction of viable cell numbers or improvement of ethanol yield with higher concentrations of THIAA. Concentrations above 12 ppm might promote resistance of *Lb. brevis* to THIAA. Figure 4 shows the effective concentration of THIAA for inhibition of *Lb. fermentum* was about 3 ppm. The optimum concentration at which all *Lb. fermentum* were killed was about 6 ppm.

Figure 5 shows the effective concentration of HHIAA for inhibition of *Lb. brevis* was about 4 ppm. The optimum concentration at which viable cell numbers were extensively reduced was about 10 ppm.

Figure 6 shows the effective concentration of HHIAA for inhibition of *Lb. fermentum* was about 4 ppm. The optimum concentration at which all cells were killed was about 8 ppm. There was no improvement in reduction of viable cell numbers or improvement of ethanol yield with higher concentrations of HHIAA.

Figure 7 shows the effective concentration of IAA for inhibition of *Lb.s brevis* was about 6 ppm. The optimum concentration at which all cells were killed was about 12 ppm. Figure 8 shows that the effective concentration of iso-a- acids for inhibition of *Lb. fermentum* was about 4 ppm. The optimum concentration at which all cells were killed was about 8 ppm. Concentrations as high as 20 ppm of IAA showed an improvement in ethanol yield which might be due to stress of yeast.

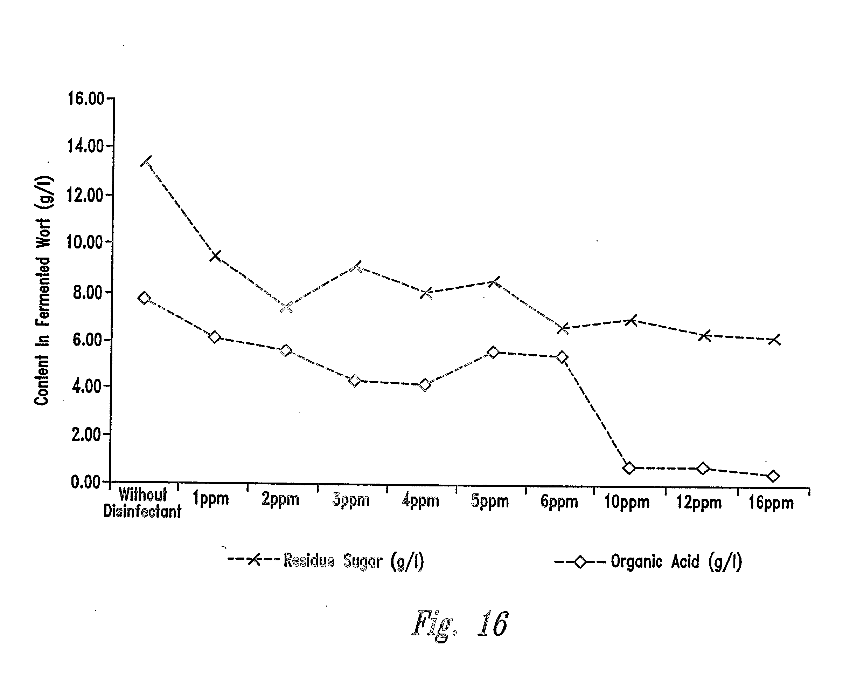

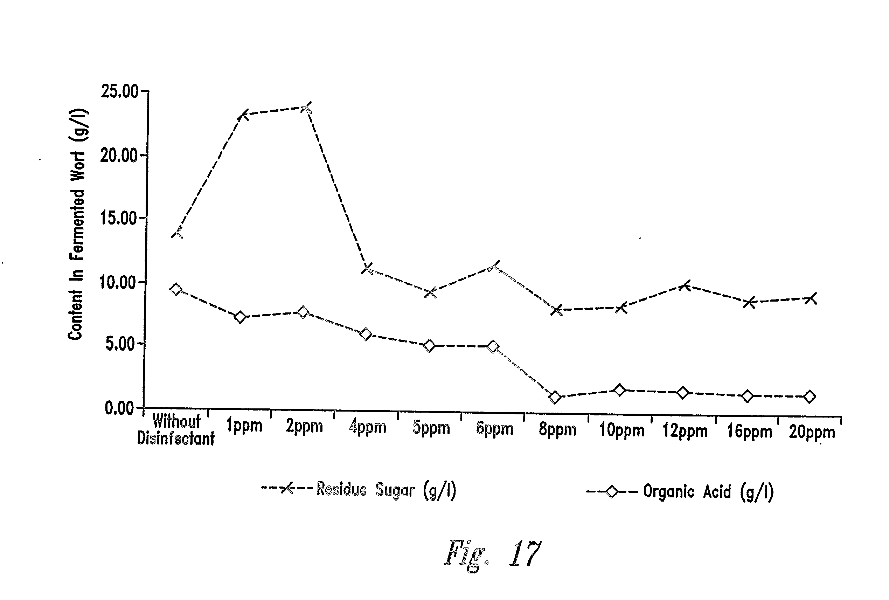

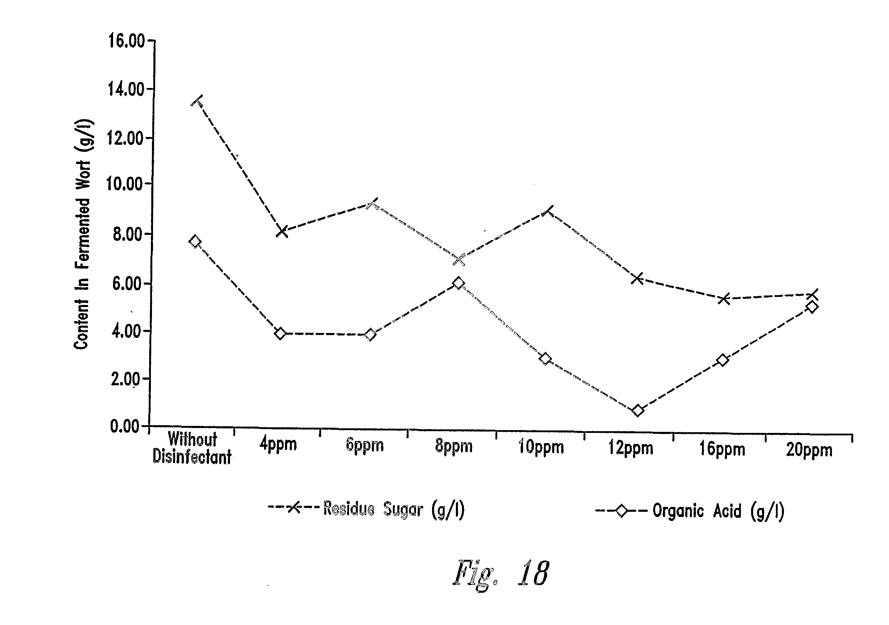

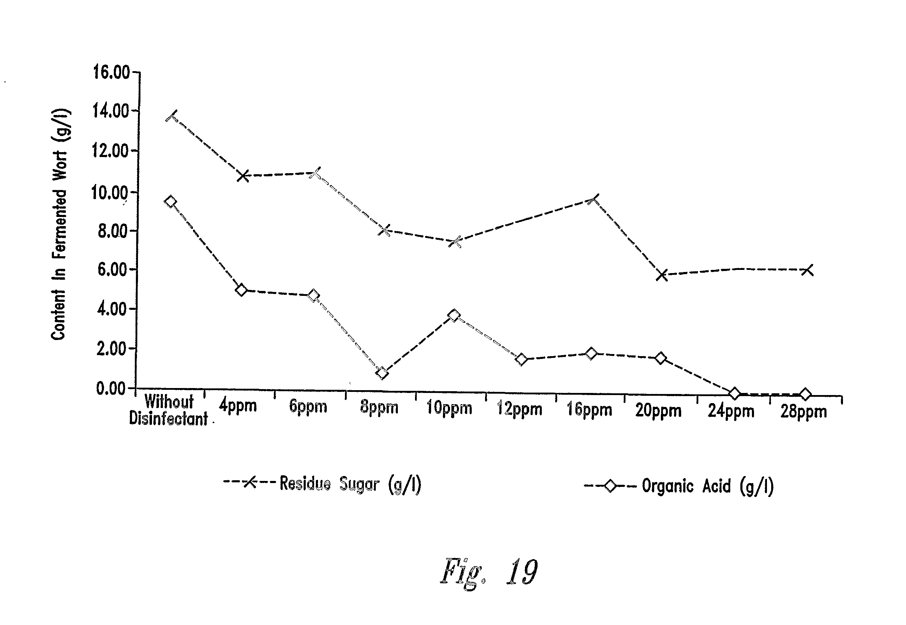

In the case of IAA, the effective concentrations from the fermentation tests and the MIC concentrations correlated with the optimum concentrations. Figures 9-14 shows the decrease of bacterial metabolites produced by *Lb. brevis* and *Lb. fermentum* at increasing concentrations of hop acids. *Lb. brevis* and *Lb. fermentum* are both strains of heterofermentative bacteria and produce lactic acid, acetic acid, ethanol and $CO_2$. Numbers of *Lb. fermentum* in sugar beet molasses wort contaminated with $10^6$ CFU/mL (without disinfectant) reached $10^9$/mL, produced more lactic acid and acetic acid and provoked heavier losses in ethanol yield than *Lb. brevis. Lb. brevis* grew slower and reached cell numbers of $5 \times 10^7$. Figures 15- 19 show the run of the decreasing curve of residue sugar (i.e. raffinose, sucrose, glucose, and fructose) in fermented wort was synchronized to that of organic acids.

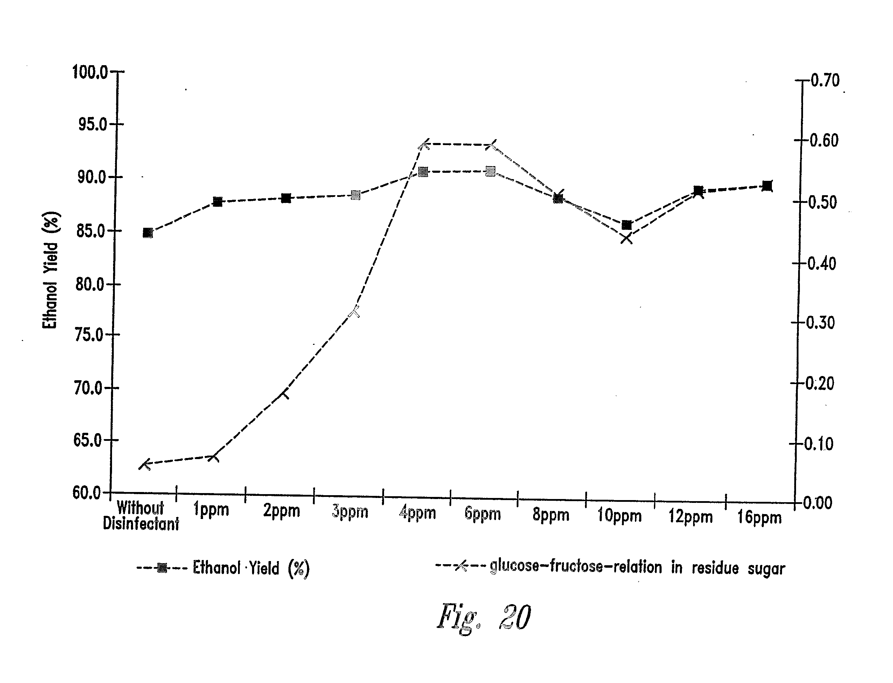

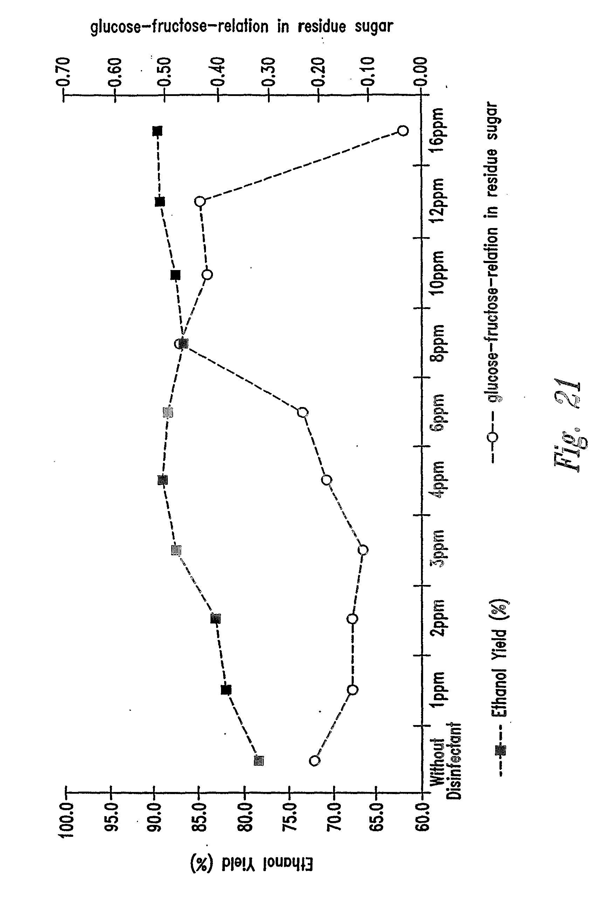

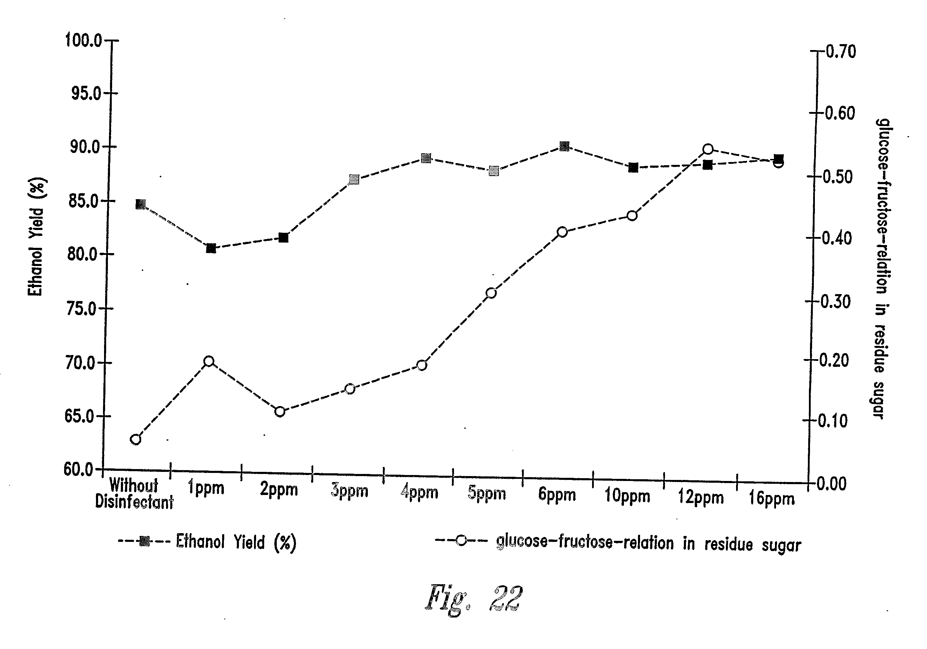

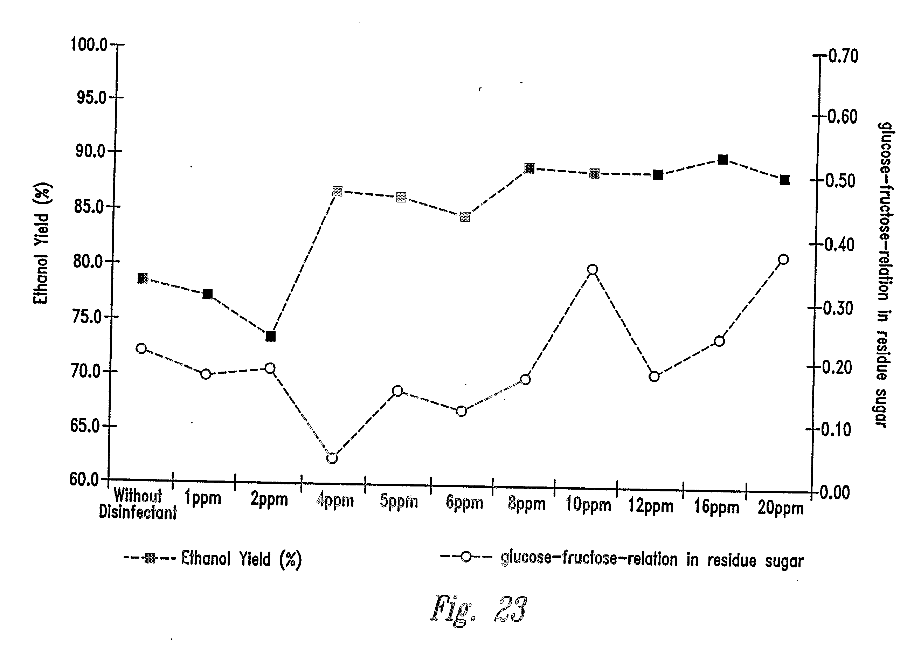

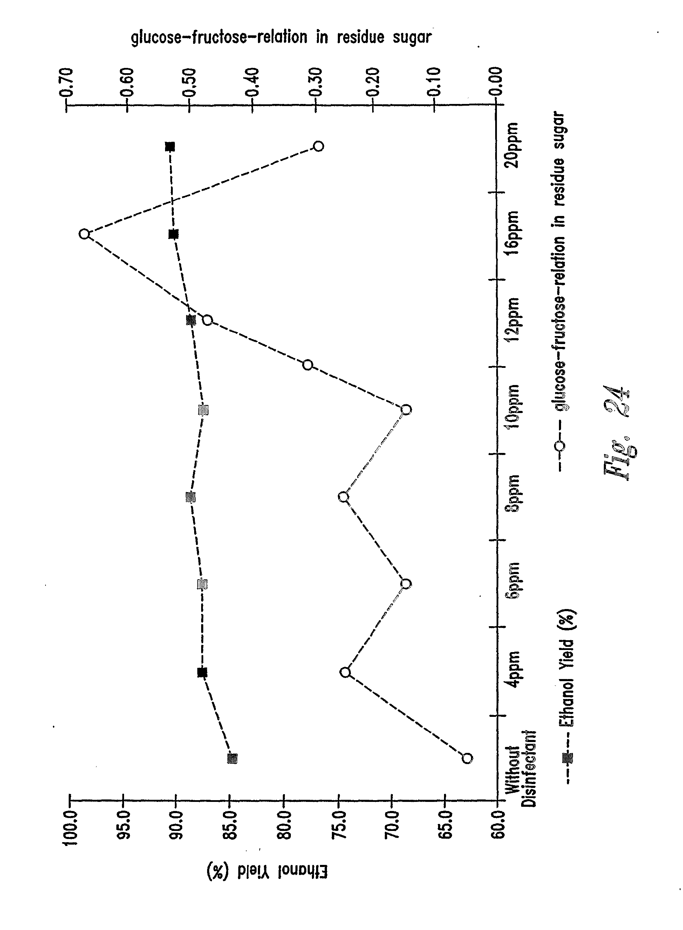

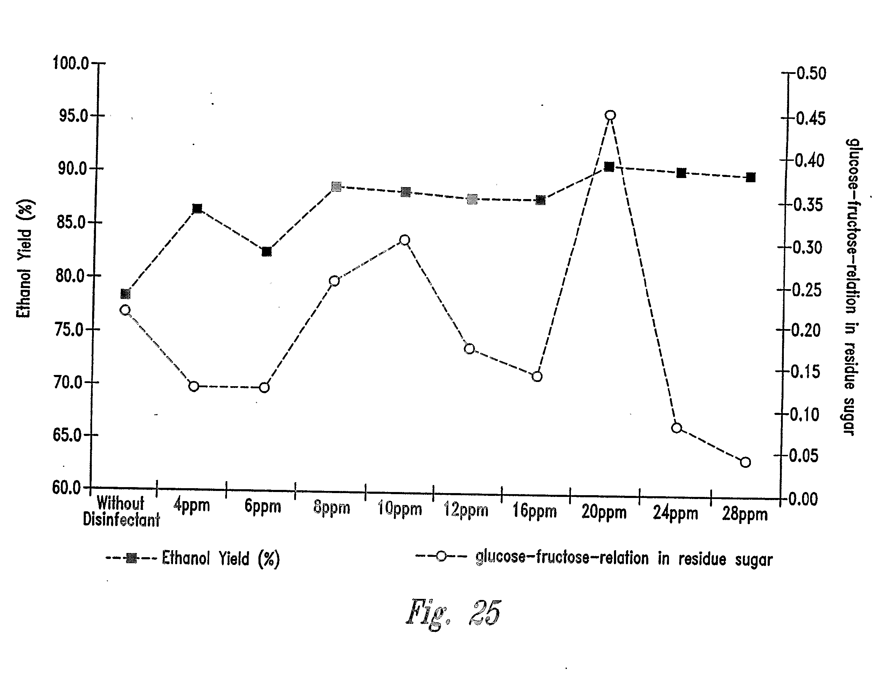

Figures 20-25 illustrate the influence of the glucose-fructose-relation in residue sugar at increasing concentrations of THIAA, HHIAA, and IAA. Good ethanol yields are generally achieved at a relation greater than 0.2.

**Example 3: Properties of Iso-$\alpha$-acids, hexahydro-iso-a-acids and tetrahydro-iso-$\alpha$-acids compared to conventional antibiotics in molasses wort when inoculated with $10^6$ CFUI mL of *Lactobacillus brevis* or *Lactobacillus fermentum***

The results of the fermentation experiments with hop acids were compared to the results of fermentation experiments using the conventional antibiotics Penicillin G and Virginiamycin as disinfectants.

Penicillin is often used over 1.5 ppm in batch fermentations due to the possibility of induced enzymatic degradation of this antibiotic by some bacteria and the rather poor stability of penicillin G below pH 5 (Kelsall 1995). In this case, 0.25 ppm penicillin G was used, according to the manufacturer's instruction.

0.5 ppm of Virginiamycin was used. Virginiamycin at a concentration of 0.5 ppm is effective against most lactic acid bacteria (Hynes S.H. et. al., J. Ind. Micro. & Biotech; 18 (4) : 284- 291, 1997.) The worts were identically inoculated with $10^6$ CFU/ mL of *Lb. brevis* or *Lb. fermentum.*

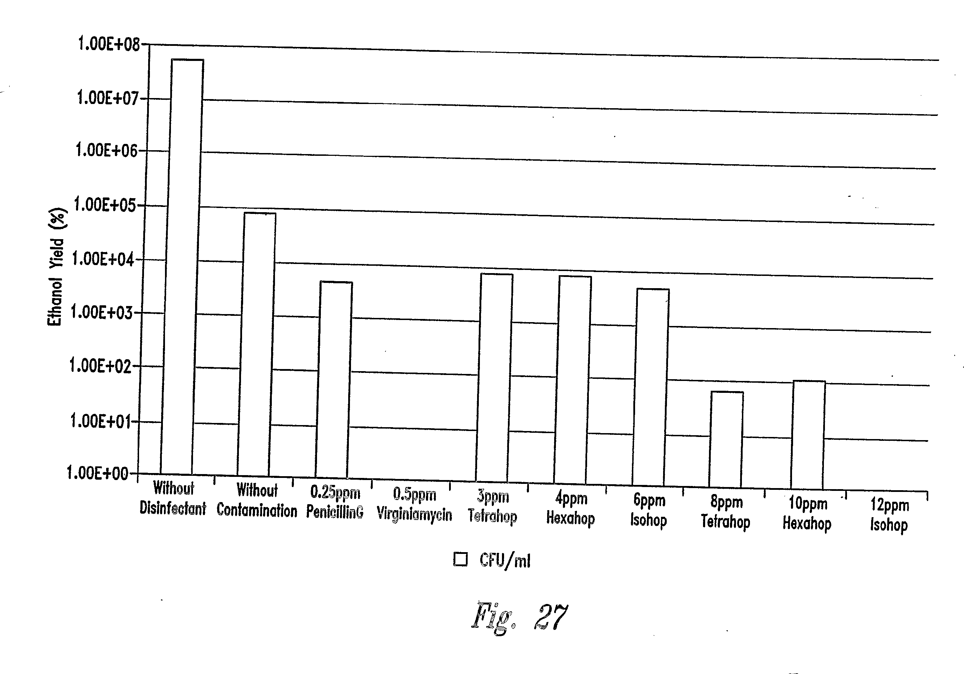

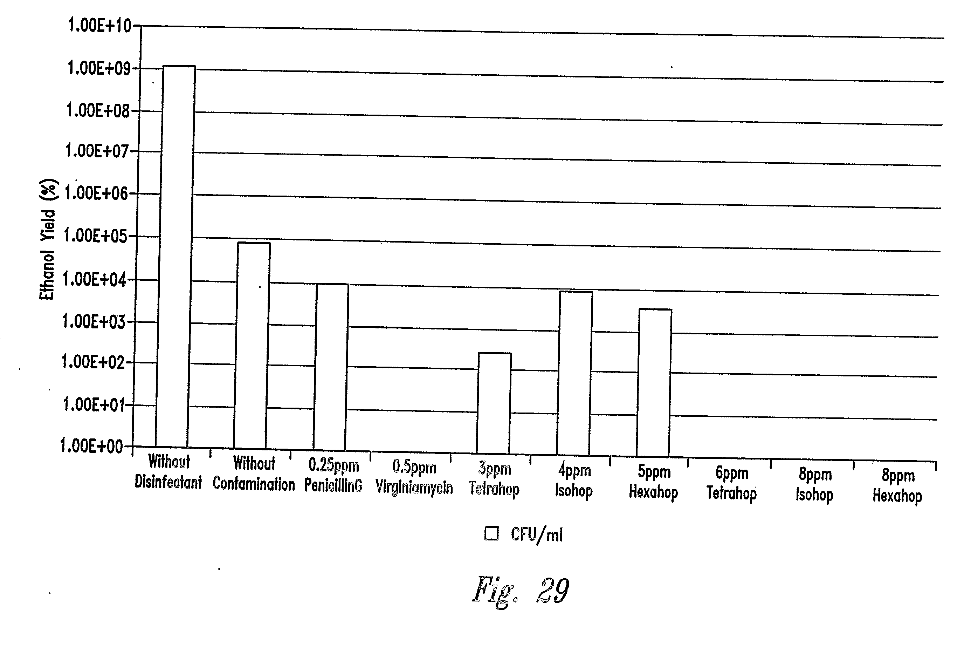

Ethanol yields (Figs. 26 and 28) and viable cell numbers (Figs. 27 and 29), which were achieved with both antibiotics, were compared to the ethanol yields in undistatbed fermentations without hop acids and to the ethanol yields of each effective and optimum concentration of IAA and their derivates. Both effective and optimum concentrations of each hop acid gave better ethanol yields than were achieved with penicillin G or Virginiamycin. All contaminated worts, where growth of lactic acid bacteria had been successfully inhibited achieved better ethanol yields than worts without deliberate contamination.

Virginiamycin was most effective against bacteria in all tests, leaving no viable cells. The effective concentrations of hop acids reduced bacteria count in a dimension similar to Penicillin G. The optimum concentrations were as effective as Virginiamycin in case of *Lb. fermentum.*

**Example 4: Properties of Iso-$\alpha$-acids, hexahydro-iso-a-acids and tetrahydro-iso-$\alpha$-acids in wheat mash**

In all fermentation experiments with wheat mash medium, lactic acid bacteria were harvested by centrifugation and inoculated as concentrated cell suspension in 0.85 saline solution after washing twice with sterile 1 % peptone water. Appropriate quantities were added to wheat mash to give initial viable cell numbers of $10^7$/mL. Wheat mash contained 15.7 % solids.

Growth and lactic acid production by the bacteria was not sufficient to have a vast effect on ethanol yield. In samples which contained no inhibitory substance at all, growth and lactic acid production provoked losses in ethanol yield up to 7 %. The observed losses in ethanol yield were greater than expected losses calculated from the amount of glucose diverted for the production of lactic acid. Even minimal concentrations of hop acids below the MICs stopped growth of bacteria and widely reduced the production of organic acids, although the reduction of viable cell numbers below $10^4$/ mL required concentrations of hop acids high above the MICs. This is certainly not only related with the higher inoculation of bacteria, but also with the higher viscosity of wheat mash and the better nutritive situation for lactobacilli in wheat mash. Again *Lb. fermentum* grew faster than *Lb. brevis* and produced higher amounts of organic acid, but was more sensitive towards hop acids.

Not enough lactic acid was produced to disturb sugar consumption by yeast. Other than in the test series with sugar beet molasses wort, the amounts of residue sugar, consisting of maltose, glucose and fructose remained constant and rather increased with reduced viable cell numbers. The glucose-fructose relation' was not essentially affected and was 0.5 or higher.

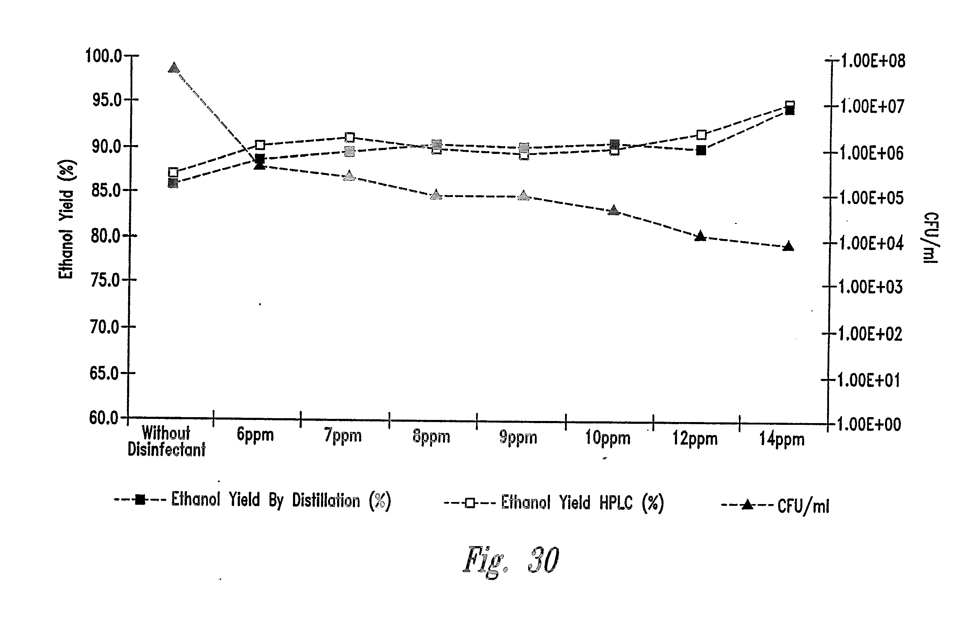

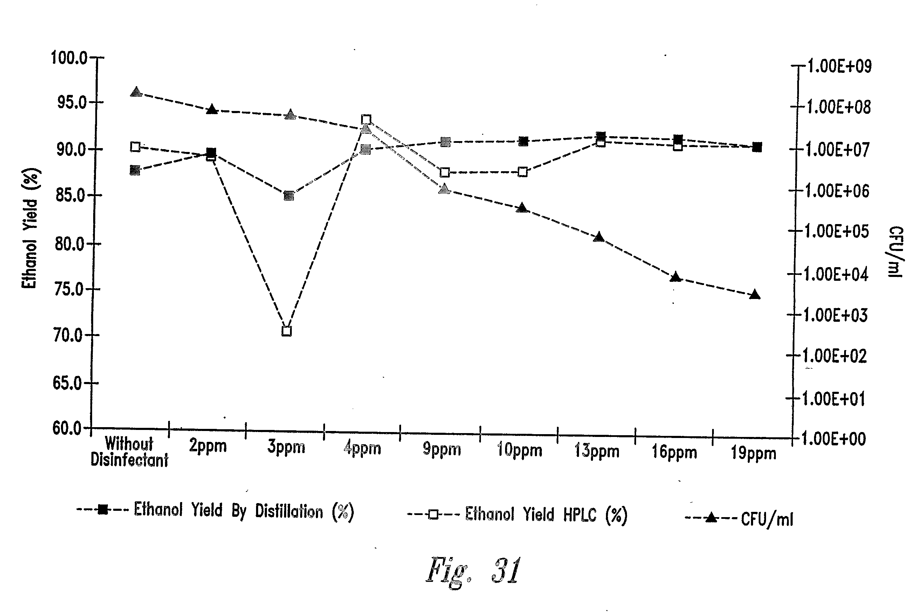

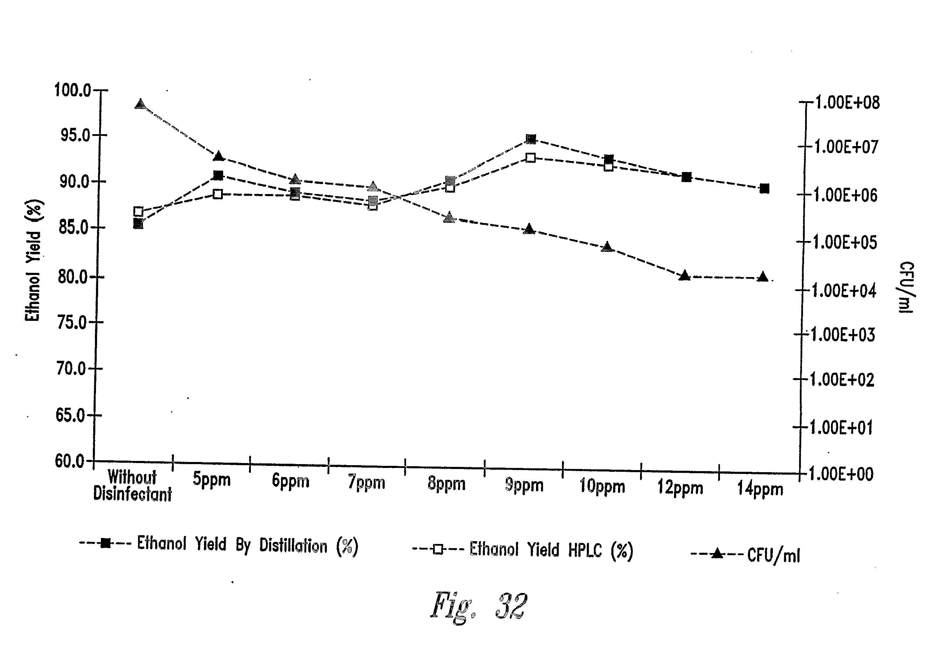

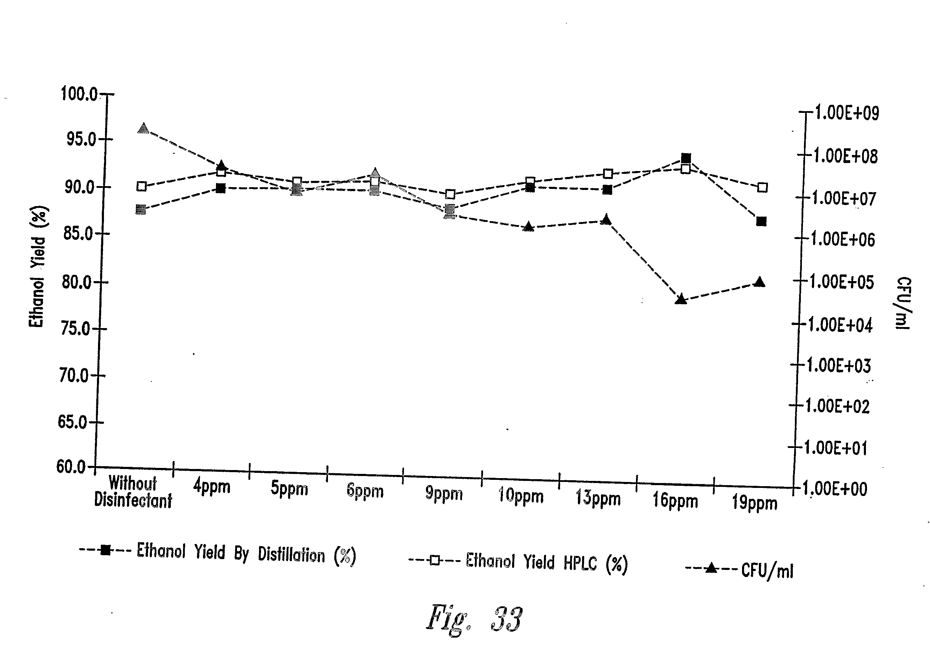

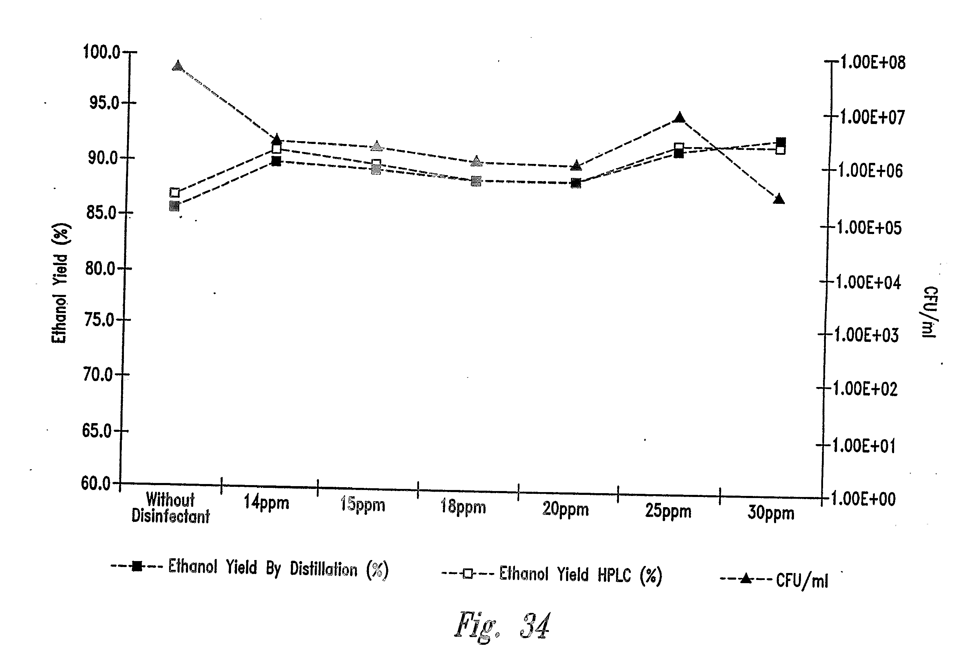

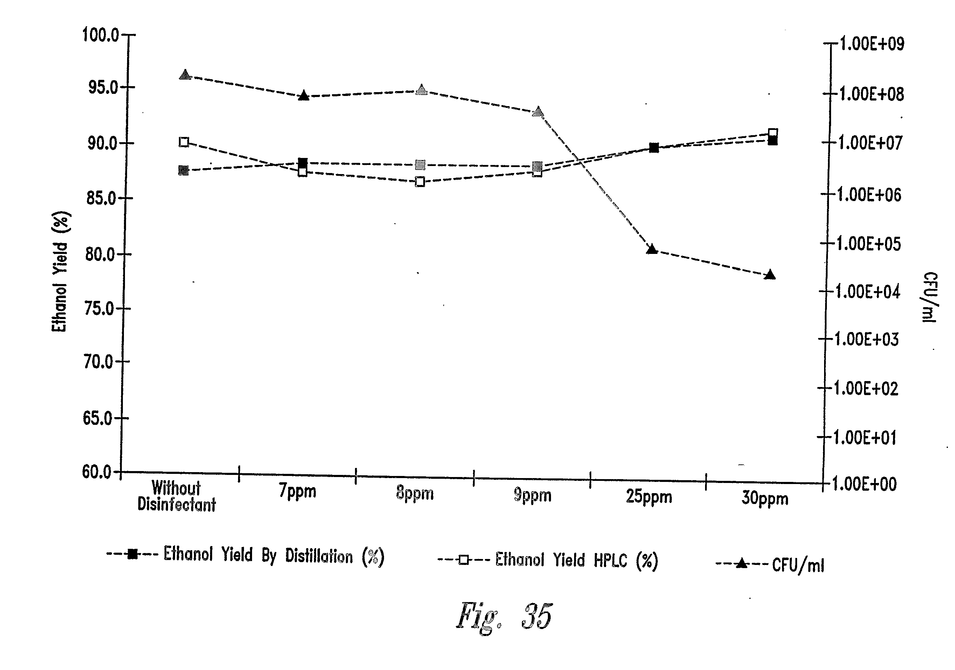

The effective concentration of THIAA, shown in Figures 30 and 31, and HHIAA, shown in Figures 32 and 33, for inhibition of *Lb. brevis* and *Lb. fermentum* was about 14-16 ppm. As shown in Figures 34 and 35, the effective concentration of IAA for inhibition of *Lb. brevis* and *Lb. fermentum* was above 30 ppm.

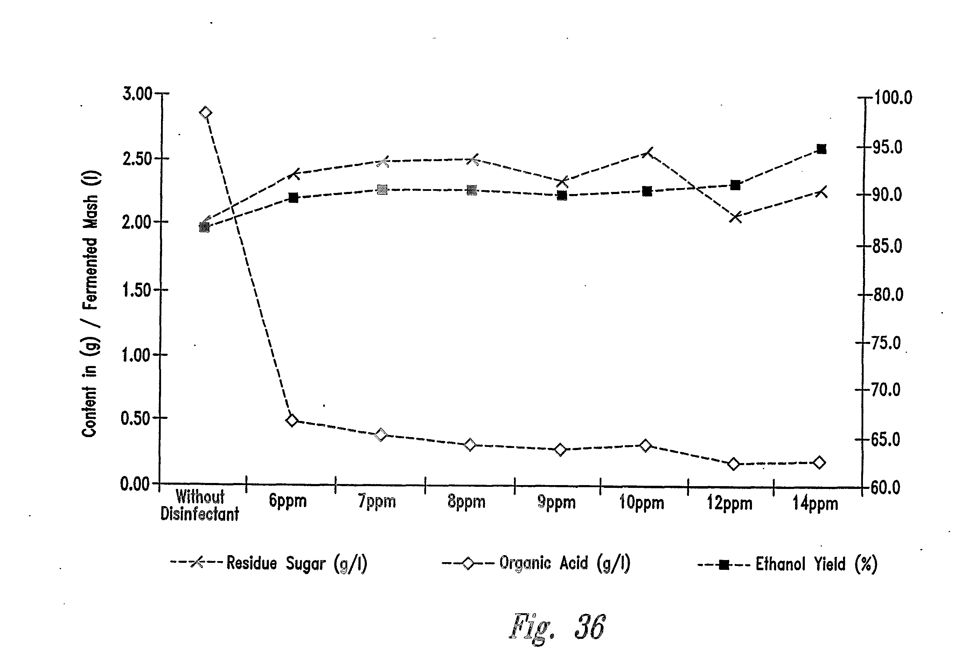

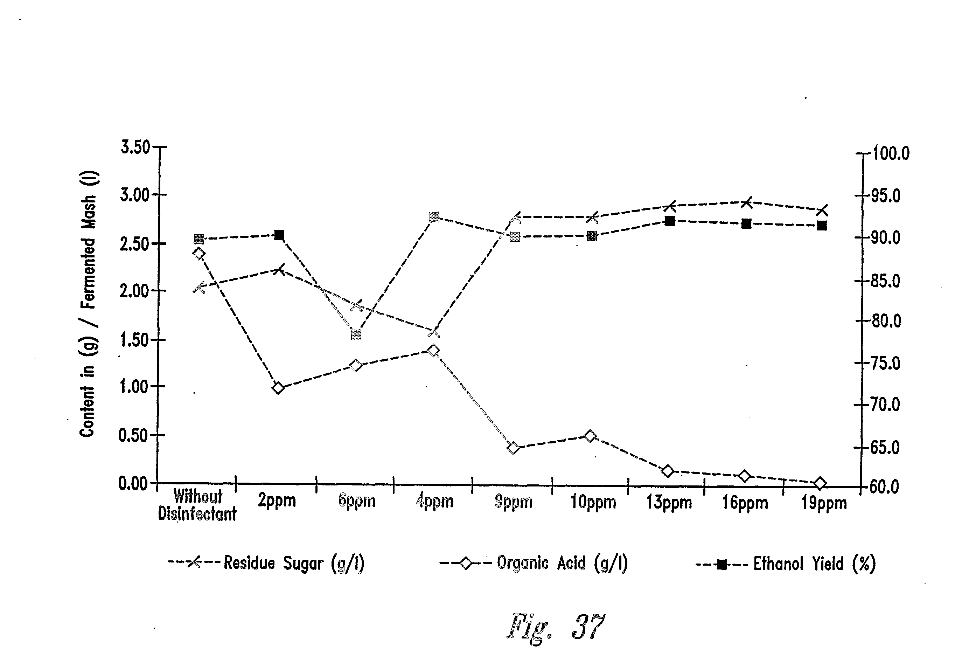

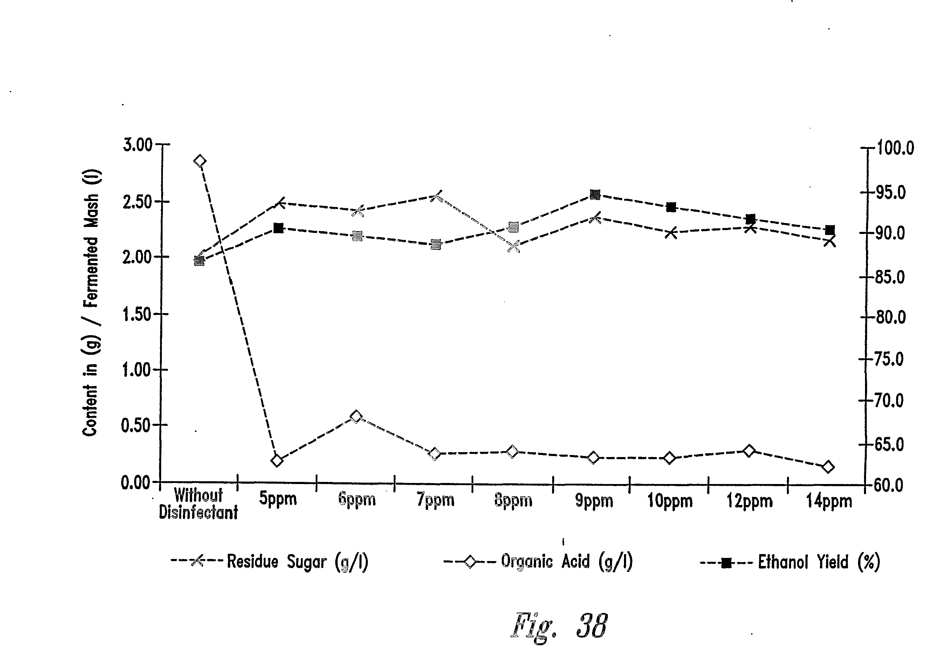

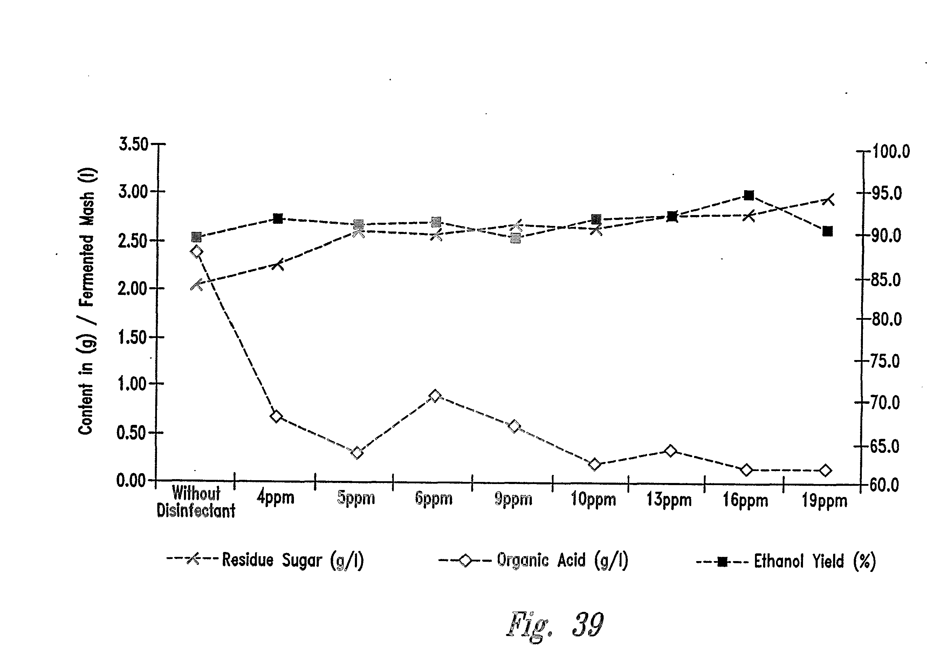

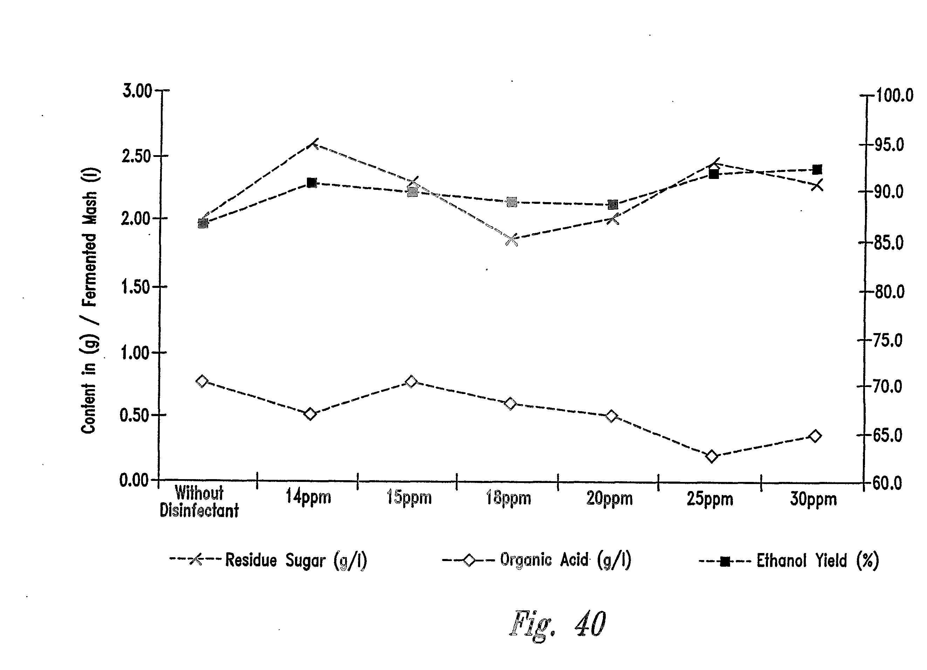

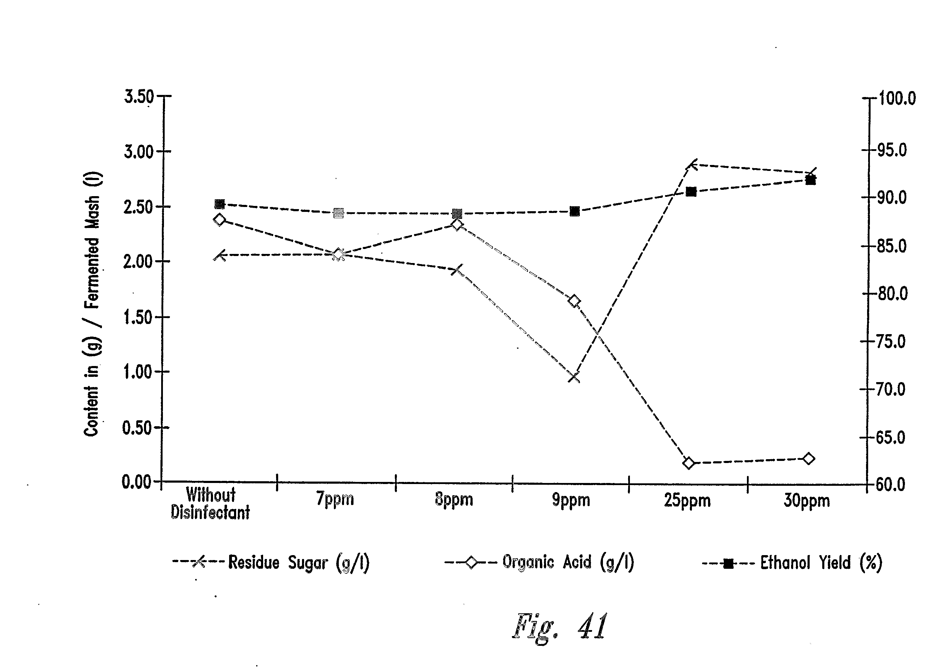

Figs. 36- 41 shows the development of ethanol yield, content of residue sugar and bacteria metabolites at decreasing viable cell numbers of *Lb. brevis* or *Lb. fermentum* correlated with increasing concentrations of hop acids in wheat mash.

**Example 5: Properties of Iso-α-acids, hexahydro-iso-α-acids, and tetrahydro-iso-α-acids compared to conventional antibiotics in molasses wort when inoculated with $10^7$ CFU/ mL of *Lactobacillus brevis* or *Lactobacillus fermentum***

The results of the fermentation experiments with hop acids were compared to the results of fermentation experiments using the conventional antibiotics Penicillin G and Virginiamycin as disinfectant.

0.25 ppm Penicillin G was used, according to the manufacturer's instruction and 0.5 ppm of Virginiamycin was used. The worts were identically inoculated with $10^7$ CFU/mL of *Lb. brevis* respectively *Lb. fermentum.*

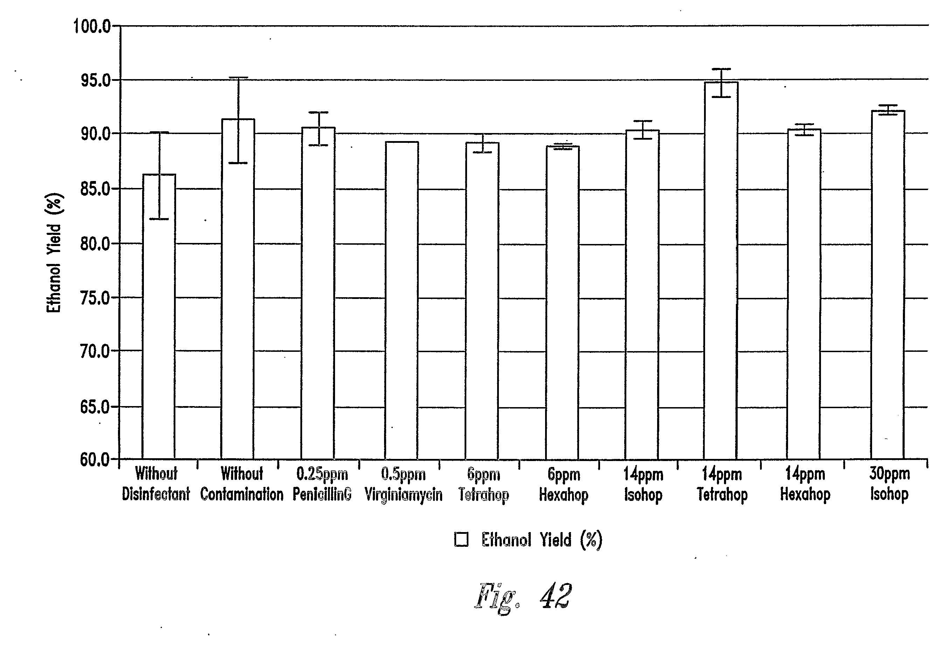

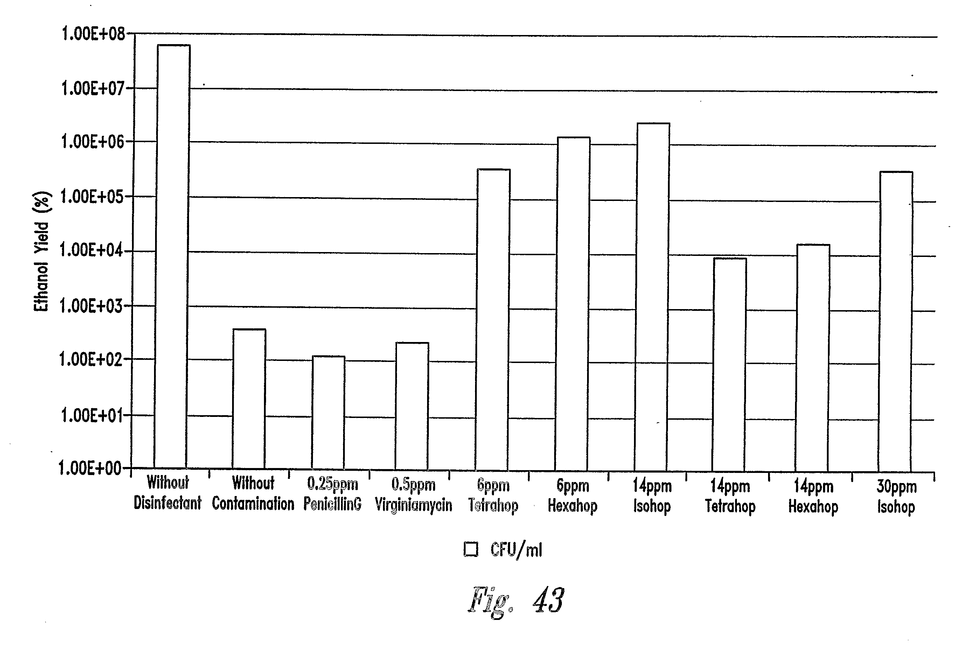

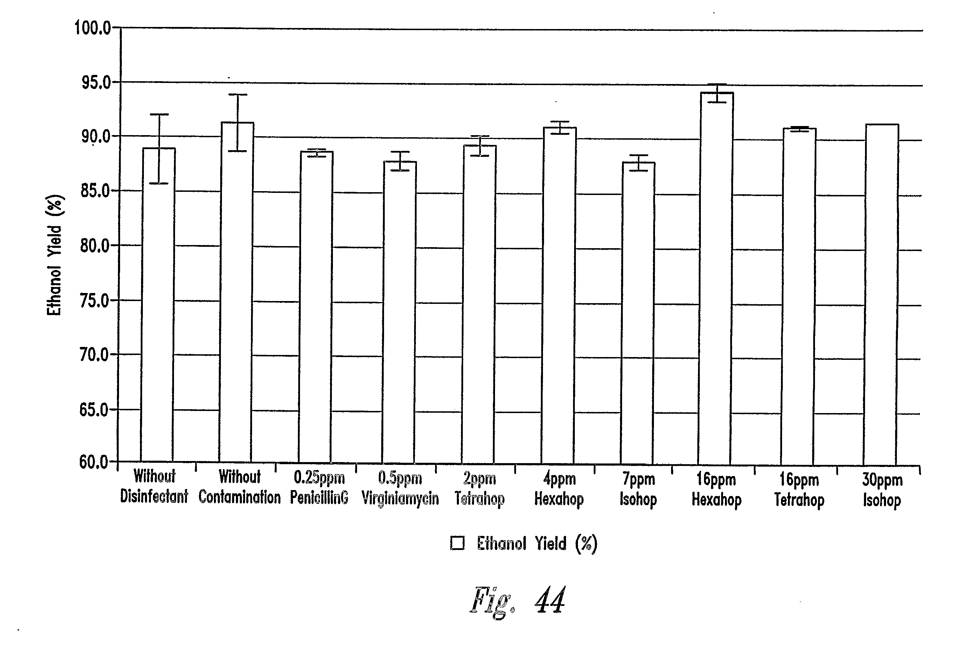

Ethanol yields (Figs. 42 and 44) and viable cell numbers (Fig. 43), which were achieved with both antibiotics, were compared to the ethanol yields in undisturbed fermentations without disinfectant and to the ethanol yields of each effective and optimum concentration of IAA and their derivates. Both minimal and effective concentrations of each hop acid gave similar or better ethanol yields than were achieved with Penicillin G or Virginiamycin. Effective concentrations achieved similar or better ethanol yields than worts without deliberate contamination. In worts contaminated with *Lb. brevis* Penicillin G and Virginiamycin reduced viable cell numbers below $10^3$/mL and below viable cell numbers in worts without contamination. The effective concentrations of Tetrahop™ Gold and Hexahop Gold™ reduced viable cell numbers to $10^4$.

In worts contaminated with *Lb. fermentum,* Virginiamycin was most effective and reduced viable cells to $10^3$ cells/mL. The use of Penicillin G showed practically no effect. The effective concentrations of Tetrahop™ Gold Hexahop Gold™ and Isohop reduced viable cell numbers to approximately $10^4$ cells/mL.

**Example 6**

An alkaline solution of isoalpha acid is dosed to the fermentation stage of a distillery in a concentration of about 10 to about 20 ppm. The temperature of the fermentation stage is below 30°C and the pH is below 6.

**Example 7**

Two peristaltic pumps were calibrated using deionized water to deliver 20 ppm of isoalpha acids to two 28°C molasses streams. One pump dosed ISOHOP® (a 30 wt. % aqueous solution of potassium salt isoalpha acid commercially available from Haas Hop Product, Inc.) to a dilute molasses stream, 20 brix (20 % solids) feeding three yeast growing tanks. The other pump dosed ISOHOP® to a dilute molasses stream, 26 brix, feeding the 8 fermentors. These two streams ran constantly and the distillery ran essentially semicontinuous. Dip-tubes and valves were welded to the two pipes which delivered these two molasses streams.

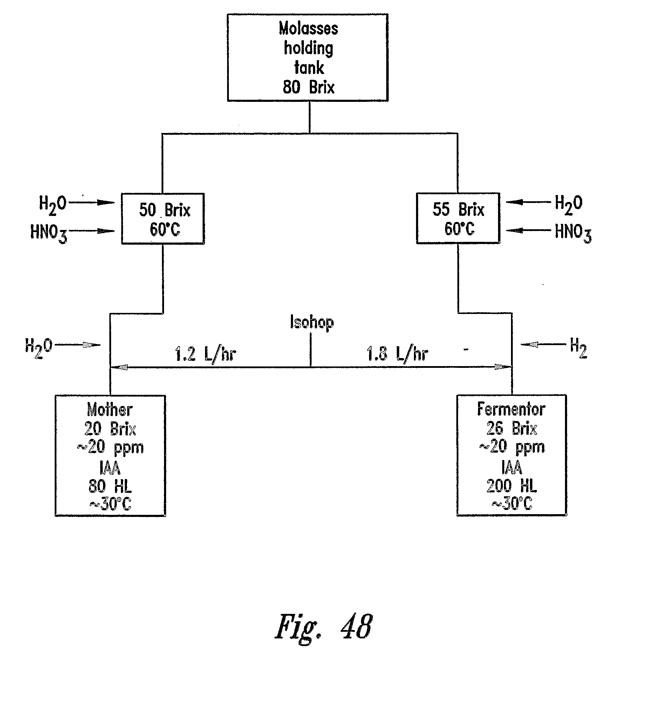

Figure 48 is a diagram showing how the concentrated molasses is first diluted to about 50 to about 55 brix and pH adjusted to about 6.2 at 60°C. The dilutions took about 45-60 minutes and were further diluted downstream and cooled to 30°C prior to  ISOHOP® addition and introduction into the yeast growing tank and the fermentor. The concentrated molasses contains some bacteria, however, at 80 brix there is not enough water for the bacteria to grow, therefore, it remains dormant. Once diluted, however, the bacteria has an opportunity to grow. Therefore, ISOHOP® was introduced into the diluted molasses solution as soon as possible. Because the dilution tanks were small, dilutions were constantly being performed and sent forward to their appropriate tanks. It takes about 4 hours to fill each yeast growing tank, about 16 hours to fill the fermentation tank with molasses and fermentation took an additional 48 hours.

The yeast growing solution from the yeast growing tank and the "wine" from the fermentation were loaded with lactobacillus. Analytical analysis showed the bacteria count to be 3 million bacteria cells/mL. These two solutions were also analyzed for residual sugar, alcohol yield and total organic acids, such as lactic acid, acetic acid etc.

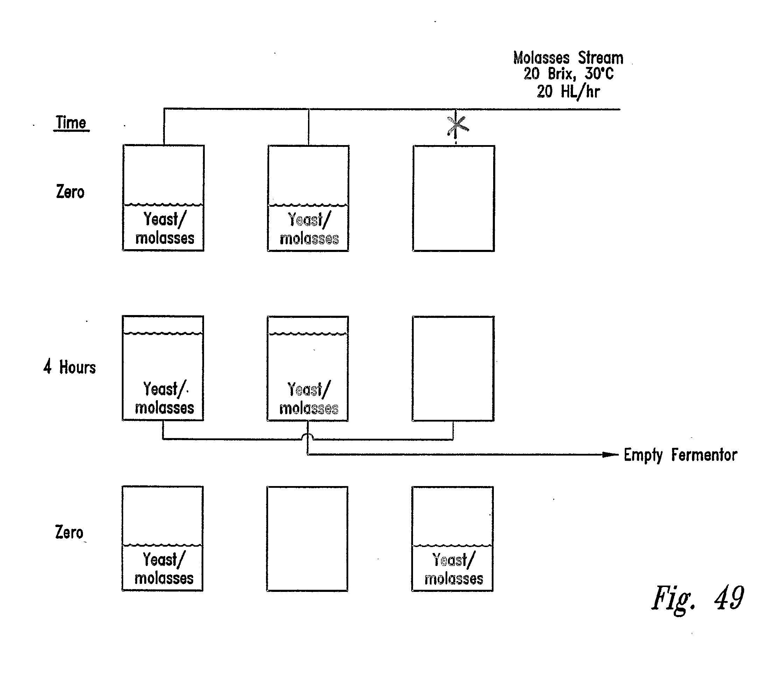

Figure 49 is a diagram demonstrating the growth of yeast in the yeast growing tanks. At time zero there were two yeast growing tanks which hold a total volume of 100 HL each. Each tank contained about 40 HL of yeast and molasses feed and was constantly aerated. The molasses feed was constantly added to two yeast growing tanks at a flow rate of 20 HL per hour. It takes four hours to fill these two tanks to a volume of 80 HL each. After each tank reached a total volume of 80 HL, one tank was transferred to an empty fermentor while half of the other tank was pumped into the third empty yeast growing tank to continue the process of growing more yeast.

After the 80 HL of yeast solution was sent to an empty fermentor 120 HL of molasses - 26 brix was added to this fermentation tank. The addition of this molasses solution took about 16 hours and 48 hours after molasses addition the fermentation was complete. The combined 200 HL of molasses/yeast/alcohol etc was pumped to the distillation towers to isolate the ethanol.

After dosing for about 20 hours 15 ppm of ISOHOP® was added to the molasses feed going into the fermentor and about 13 ppm of ISOHOP® was added to the molasses feeding the yeast growing solution. Microscopic inspection of the yeast growing solution and fermentation solutions indicated a lowering of the bacteria.

40 hours after dosing it was clear that the bacteria count in the yeast growing solution was down significantly and the fermenting solution looked about normal. The first fermentation with ISOHOP® was complete. Samples of the wine were analyzed which showed that the amount of organic acid was reduced by about 0.4% vs. before ISOHOP® addition. The residual sugar in the wine measured 130 ppm and distillation of this material produced a normal ethanol yield. The yeast cells in the fermentor showed no flocculation indicating that bacteria contamination was low.

After three days of dosing 11 ppm of ISOHOP® into the yeast growing solution and 15 ppm into the fermentor, microscopic inspection of the yeast growing solution showed little to no lactobacillus bacteria and the fermentation solutions looked normal. Based on the fact that the antibiotic Virginiamycin reduces the bacteria count by only 50% it appears that ISOHOP® works better than Virginiamycin.

On day four dosing of ISOHOP® into the fermentor stopped and 11 ppm of ISOHOP® was dosed into the yeast growing tank for the next 48 hours. This 11 ppm solution was diluted to 4 ppm once the molasses solution was added to the fermentor. Analysis of the yeast growing solution showed little to no lactobacillus and only few cocci bacteria and the fermentor solutions showed little to no difference between those fermentations which had 15 ppm of ISOHOP® and those currently receiving 4 ppm ISOHOP® via the yeast growing tanks.

The discussion above is descriptive, illustrative and exemplary and is not to be taken as limiting the scope defined by any appended claims.

**Claims**

1. A method for controlling lactic acid bacteria contamination in a process medium used in the production of fuel ethanol comprising:

   adding an aqueous alkaline solution of hop acid to a process medium having a pH less than the pH of the alkaline hop acid solution,
   wherein the hop acid is selected from at least one of the group consisting of alpha acids, beta acids, isoalpha acids, rho-isoalpha acids, tetrahydroisoalpha acids and hexahydroisoalpha acids and salts thereof.

2. The method of claim 1 wherein the process medium is selected from the group consisting of claim a yeast propagation tank, a fermentation tank, a steep tank and a starch/glucose stream in the dry milling process and wet milling process.

3. The method of claim 1 further comprising adding a minimum inhibitory concentration of hop acid into the process medium.

4. The method of claim 1 wherein the concentration of hop acid is about 1 ppm to about 30 ppm.

5. The method of claim 4 wherein the hop acid is selected from at least one of the group consisting tetrahydroisoalpha acid and hexahydroisoalpha acid and salts thereof and the concentration is about 2 ppm.

6. The method of claim 4 wherein the hop acid is isoalpha acid and salts thereof and the concentration is about 4 ppm.

7. The method of claim 1 wherein the concentration of hop acid is isoalpha acids, tetrahydroisoalpha acids and hexahydroisoalpha acids or salts thereof and the concentration to control bacteria in a fermentable solution are above 12 ppm, 8 ppm and 10 ppm respectively.

8. The method of claim 1 wherein the hop acid is added into the process medium discontinuously.

9. The method of claim 1 wherein the hop acid is added to the process medium by shock dosage.

10. The method of claim 1 wherein the hop acid is added to the process medium continuously.

11. The method of claim 1 wherein the process medium is selected from the group consisting of a yeast propagation tank and a fermentation tank and
    wherein said hop acid is added into the yeast propagation tank and following the yeast growth, the yeast solution containing hop acid is transferred to the fermentation tank.

12. The method of claim 1 wherein said hop acid is selected from at least one of the group consisting of isoalpha acids, tetrahydroisoalpha acids and hexahydroisoalpha acids and salts thereof.

*Fig. 1*

Fig. 2

Fig. 3

*Fig. 4*

Fig. 5

EP 2 650 355 A1

Fig. 6

Fig. 7

27

Fig. 8

Fig. 9

*Fig. 10*

EP 2 650 355 A1

Fig. 11

EP 2 650 355 A1

Fig. 12

EP 2 650 355 A1

Fig. 13

*Fig. 14*

*Fig. 15*

*Fig. 16*

Fig. 17

*Fig. 18*

*Fig. 19*

--- ▦ --- Ethanol·Yield (%)          --- ✕ --- glucose-fructose-relation in residue sugar

*Fig. 20*

*Fig. 21*

EP 2 650 355 A1

Fig. 22

Fig. 23

Fig. 24

Fig. 25

*Fig. 26*

EP 2 650 355 A1

*Fig. 27*

Fig. 28

*Fig. 29*

Fig. 30

Fig. 31

Fig. 32

EP 2 650 355 A1

Fig. 33

Fig. 34

EP 2 650 355 A1

Fig. 35

--✕-- Residue Sugar (g/l)  ⎯◇⎯ Organic Acid (g/l)  ---■--- Ethanol Yield (%)

*Fig. 36*

EP 2 650 355 A1

Fig. 37

EP 2 650 355 A1

Fig. 38

EP 2 650 355 A1

Fig. 39

EP 2 650 355 A1

*Fig. 40*

- - -✗- - - Residue Sugar (g/l)    - - -◇- - Organic Acid (g/l)    - - -■- - - Ethanol Yield (%)

EP 2 650 355 A1

*Fig. 41*

*Fig. 42*

EP 2 650 355 A1

Fig. 43

Fig. 44

*Fig. 45*

EP 2 650 355 A1

```
                          ┌─────────────────┐
                          │   Heat Water    │
                          │  to 70–75°C.    │
                          └────────┬────────┘
                                   │
                                   ▼
┌─────────────────┐      ┌─────────────────┐      ┌─────────────────┐
│ Aqueous solution│─────▶│      Mix.       │◀─────│ Addition of liquid│
│    of KOH.      │      │                 │      │    beta–acid.   │
└─────────────────┘      └────────┬────────┘      └────────┬────────┘
                                   │                        │
                                   ▼                        ▼
                          ┌─────────────────┐      ┌─────────────────┐
                          │Maintain temperature│    │ Adjusting addition│
                          │ for 15–30 minutes.│    │  of beta–acid to │
                          └────────┬────────┘      │   reach final   │
                                   │               │ concentration of │
                                   ▼               │     5–10%.      │
                          ┌─────────────────┐      └─────────────────┘
                          │ Mixture separates│
                          │ into clear, basic│
                          │ solution and turbid,│
                          │  oil–containing │
                          │   components.   │
                          └────────┬────────┘
                                   │
                                   ▼
                          ┌─────────────────┐      ┌─────────────────┐
                          │ Separate clear, │      │  Cool to 2–7°C. │
                          │ basic, beta–acid│─────▶│                 │
                          │  solution by    │      └────────┬────────┘
                          │ adjusting pH to │               │
                          │    10–10.5.     │               ▼
                          └─────────────────┘      ┌─────────────────┐
                                                   │  Discontinuous  │
┌─────────────────┐                                │addition to process│
│ Conversion from │◀───────────────────────────────│     medium.     │
│ dissociated to  │                                └─────────────────┘
│ undissociated form│
│ of beta–acid salts│◀─ ─ ─ ─ ─ ─ ─ ─ ─ ─ ─ ┐
│  in process     │                         ╎
│    medium.      │                         ╎
└─────────────────┘              ┌─ ─ ─ ─ ─ ─ ─ ─ ─ ┐
                                 ╎ Further addition of╎
                                 ╎ alkaline base if  ╎
                                 ╎    needed.        ╎
                                 └─ ─ ─ ─ ─ ─ ─ ─ ─ ┘
```

## Fig. 46

Fig. 47

*Fig. 48*

Molasses Stream
20 Brix, 30°C
20 HL/hr

**Time**

Zero

Yeast/
molasses    Yeast/
molasses

4 Hours

Yeast/
molasses    Yeast/
molasses

→ Empty Fermentor

Zero

Yeast/
molasses        Yeast/
molasses

*Fig. 49*

Europäisches
Patentamt

European
Patent Office

Office européen
des brevets

**EUROPEAN SEARCH REPORT**

Application Number

EP 13 17 5586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/52212 A1 (HAAS HOP PRODUCTS INC [US]; MAYE JOHN PAUL [US]; BEDDIE DAVID [GB]) 8 September 2000 (2000-09-08) * page 4, line 9 - line 22; claims; example 5 * | 1-12 | INV. C12C3/08 C12C3/12 C12P7/06 |
| A | US 2003/015480 A1 (BREEN ALEXANDER W [US] ET AL) 23 January 2003 (2003-01-23) * paragraphs [0047], [0096]; claims 1,2,20 * | 1 | |
| A | WO 00/65632 A2 (MILLER BREWING [US]) 2 November 2000 (2000-11-02) * page 7, lines 1-30; claims; examples * | 1 | |
| A | WO 00/53814 A1 (BETATEC HOPFENPRODUKTE GMBH [DE]; ZUCKERFORSCHUNG TULLN GMBH [AT]; MAY) 14 September 2000 (2000-09-14) * page 2, lines 31-34; claims; examples * | 1 | |
| A | US 5 082 975 A (TODD JR PAUL H [US] ET AL) 21 January 1992 (1992-01-21) * column 7, line 64 - column 8, line 13 * | 1 | TECHNICAL FIELDS SEARCHED (IPC) A23L C12C C12H C13B |
| A | SIMPSON W J ET AL: "FACTORS AFFECTING ANTIBACTERIAL ACTIVITY OF HOP COMPOUNDS AND THEIR DERIVATIVES", JOURNAL OF APPLIED BACTERIOLOGY, BLACKWELL PUBLISHING LTD., OXFORD, GB, vol. 72, no. 4, 1 January 1992 (1992-01-01), pages 327-334, XP000578432, ISSN: 0021-8847 * the whole document * | 1 | |

The present search report has been drawn up for all claims

| Place of search | Date of completion of the search | Examiner |
|---|---|---|
| Berlin | 28 August 2013 | Boeker, Ruth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**          EP 13 17 5586

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2013

| Patent document cited in search report | | Publication date | Patent family member(s) | | Publication date |
|---|---|---|---|---|---|
| WO 0052212 | A1 | 08-09-2000 | AU | 3721300 A | 21-09-2000 |
| | | | WO | 0052212 A1 | 08-09-2000 |
| US 2003015480 | A1 | 23-01-2003 | NONE | | |
| WO 0065632 | A2 | 02-11-2000 | AU | 765069 B2 | 11-09-2003 |
| | | | AU | 4800200 A | 10-11-2000 |
| | | | BR | 0009949 A | 08-01-2002 |
| | | | CA | 2367493 A1 | 02-11-2000 |
| | | | EP | 1185617 A2 | 13-03-2002 |
| | | | JP | 2002542795 A | 17-12-2002 |
| | | | MX | PA01010610 A | 04-06-2002 |
| | | | US | 6326185 B1 | 04-12-2001 |
| | | | WO | 0065632 A2 | 02-11-2000 |
| | | | ZA | 200108329 A | 10-10-2002 |
| WO 0053814 | A1 | 14-09-2000 | AT | 242818 T | 15-06-2003 |
| | | | CA | 2364880 A1 | 14-09-2000 |
| | | | CZ | 20013105 A3 | 13-03-2002 |
| | | | DE | 19909827 A1 | 07-09-2000 |
| | | | EP | 1159458 A1 | 05-12-2001 |
| | | | HU | 0200226 A2 | 29-05-2002 |
| | | | JP | 3572018 B2 | 29-09-2004 |
| | | | JP | 2003509007 A | 11-03-2003 |
| | | | PL | 351351 A1 | 07-04-2003 |
| | | | SK | 12322001 A3 | 04-04-2002 |
| | | | TR | 200102564 T2 | 21-03-2002 |
| | | | US | 6893857 B1 | 17-05-2005 |
| | | | WO | 0053814 A1 | 14-09-2000 |
| US 5082975 | A | 21-01-1992 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NARENDRANATH, N.V. et al.** *Appl. & Envir. Microbiol.,* 1997, vol. 63 (11), 4158-4163
- **ESSIA, N et al.** *Appl. Microbiol. Biotechnol.,* 1990, vol. 33, 490-493
- **HYNES S.H. et al.** *J. Indust. Microbio. and Biotech.,* 1997, vol. 18 (4), 284-291
- **BAUMGART, J.** Mikrobiologische Untersuchungen von Lebensmitteln. Behr's Verlag, Hamburg, 1994
- **HYNES S.H.** *J. Ind. Micro. & Biotech,* 1997, vol. 18 (4), 284-291